# EUROPEAN PATENT SPECIFICATION

(11) **EP 2 247 560 B1**
(45) Date of publication and mention of the grant of the patent: **04.06.2014**
(21) Application number: 09712619.7
(22) Date of filing: 19.02.2009
(51) Int. Cl.: C07C 17/383, C07C 17/386, C07C 21/18, C07C 17/38

(54) **PROCESSES FOR SEPARATION OF 1,3,3,3-TETRAFLUOROPROPENE FROM HYDROGEN FLUORIDE BY AZEOTROPIC DISTILLATION**
VERFAHREN ZUR ABSCHEIDUNG VON 1,3,3,3-TETRAFLUORPROPEN VON FLUORWASSERSTOFF MITTELS AZEOTROPER DESTILLATION
PROCÉDÉS DE SÉPARATION DE 1,3,3,3-TÉTRAFLUOROPROPÈNE DE FLUORURE D'HYDROGÈNE PAR DISTILLATION AZÉOTROPIQUE

(30) Priority: 21.02.2008 US 30371
(43) Date of publication of application: 10.11.2010
(73) Proprietor: E. I. du Pont de Nemours and Company, Wilmington, DE 19898 (US)
(72) Inventor: KNAPP, Jeffrey, P., Wilmington DE 19808 (US)
(74) Representative: Matthews, Derek Peter
(86) International application number: PCT/US2009/034492
(87) International publication number: WO 2009/105521

(56) References cited:
- WO-A-2008/008519
- WO-A-2008/024508
- US-A1- 2007 100 173

## Description

### BACKGROUND INFORMATION

### Field of the Disclosure

This disclosure relates in general to processes for separating HF from fluoroolefins.

### Description of the Related Art

The chemical manufacture of fluoroolefins may produce mixtures of the desired fluoroolefins and hydrogen fluoride (HF). The separation of fluoroolefins and HF is not always easily accomplished. Existing methods of distillation and decantation are very often ineffective for separation of these compounds. Aqueous scrubbing may be effective, but requires the use of large amounts of scrubbing solutions and produces excessive waste as well as wet product that must then be dried. Therefore, there is a need for new methods of separating HF from fluoroolefins.

### SUMMARY

In one embodiment, the present disclosure provides a process for separating a mixture comprising HF and *E*-HFC-1234ze, said process comprising a) feeding the composition comprising HF and *E*-HFC-1234ze to a first distillation column; b) removing an azeotrope composition comprising HF and *E*-HFC-1234ze as a first distillate and either i) HF or ii) *E*-HFC-1234ze as a first column bottoms composition; c) condensing the first distillate to form 2 liquid phases, being i) an HF-rich phase and ii) an *E*-HFC-1234ze-rich phase; and d) recycling a first liquid phase enriched in the same compound that is removed as the first column bottoms, said first liquid phase being either i) HF-rich phase or ii) *E*-HFC-1234ze-rich phase, back to the first distillation column.

In another embodiment, the present disclosure provides a process for separating an *E*-HFC-1234ze from a mixture comprising hydrogen fluoride and said *E*-HFC-1234ze, wherein said *E*-HFC-1234ze is present in a concentration greater than the azeotrope concentration for hydrogen fluoride and said *E*-HFC-1234ze, said process comprising a) feeding said mixture comprising hydrogen fluoride and said *E*-HFC-1234ze to a first distillation column; b) removing an azeotrope composition comprising hydrogen fluoride and *E*-HFC-1234ze as a first distillate from the first distillation column; c) recovering *E*-HFC-1234ze essentially free of hydrogen fluoride from the bottom of the first distillation column; d) condensing the azeotrope composition to form two liquid phases, being i) a hydrogen fluoride-rich phase and ii) an *E*-HFC-1234ze-rich phase; and e) recycling the *E*-HPC-1234ze-rich phase to the first distillation column.

In another embodiment, the present disclosure provides a process for separating hydrogen fluoride from a mixture comprising hydrogen fluoride and *E*-HFC-1234ze, wherein hydrogen fluoride is present in a concentration greater than the azeotrope concentration for hydrogen fluoride and said *E*-HFC-1234ze, said process comprising a) feeding said mixture comprising hydrogen fluoride and *E*-HFC-1 234ze to a first distillation column; b) removing an azeotrope or azeotrope-like composition comprising *E*-HFC-1234ze and HF as a distillate from the first distillation column; c) recovering hydrogen fluoride essentially free of *E-*HFC-1234ze from the bottom of the first distillation column; d) condensing the azeotrope composition to form two liquid phases, being an *E*-HFC-1234ze-rich phase and a hydrogen fluoride-rich phase; and e) recycling the HF-rich phase to the first distillation column.

In another embodiment, the present disclosure provides a process for the purification of an *E*-HFC-1234ze from a mixture comprising *E*-HFC-1234ze and HF, wherein said HFC-1234ze is present in said mixture in a concentration greater than the azeotrope concentration for said *E*-HFC-1234ze and HF, said process comprising a) adding an entrainer to the mixture comprising *E*-HFC-1234ze and HF thus forming a second mixture; b) distilling said second mixture in a first distillation step to form a first distillate composition comprising HF, *E*-HFC-1234ze, and entrainer, and a first bottoms composition comprising *E*-HFC-1234ze; c) condensing said first distillate composition to form two liquid phases, being i) an HF-rich phase and ii) an entrainer-rich phase; and d) optionally recycling the entrainer-rich phase back to the first distillation step.

In another embodiment, the present disclosure provides a process for the purification of HF from a mixture comprising *E*-HFC-1234ze and HF, wherein HF is present in a concentration greater than the azeotrope concentration for HF and said *E*-HFC-1234ze, said process comprising a) adding an entrainer to the mixture comprising *E*-HFC-1234ze and HF thus forming a second mixture; b) distilling said second mixture in a first distillation step to form a first distillate composition comprising an HF, entrainer, and E-HFC-1234ze, and a first bottoms composition comprising HF; c) condensing said first distillate composition to form two liquid phases, being i) an entrainer-rich phase and ii) an HF-rich phase; and d) optionally recycling the HF-rich phase back to the first distillation step.

In another embodiment, the present disclosure provides a process for the separation of *E*-HFC-1234ze from a mixture of *E*-HFC-1234ze, HF, and at least one of HFC-245fa or HFC-245eb, said process comprising: a) subjecting said mixture to a first distillation step, wherein additional *E-*HFC-1234ze is fed from a second distillation step, to form a first distillate comprising an azeotrope of *E*-HFC-1234ze and HF and a first bottoms composition comprising at least one of HFC-245fa or HFC-245eb; b) feeding said first distillate to a second distillation step to form a second distillate comprising an azeotrope of *E*-HFC-1234ze and HF and a second bottoms composition comprising *E*-HFC-1234ze essentially free of HF; c) condensing said second distillate to form two liquid phases, being i) an HF-rich phase and ii) an E-HFC-1234ze-rich phase; and d) recycling the HFC-1234ze-rich phase from (c) back to the first distillation step. In another embodiment, the present disclosure provides a process for separating HF from a mixture comprising *E*-HFC-1234ze, HF, and at least one of HFC-245fa or HFC-245eb, said process comprising a) adding an entrainer to the mixture comprising *E*-HFC-1234ze, HF, and at least one of HFC-245fa or HFC-245eb thus forming a second mixture; b) distilling said second mixture in a first distillation step to form a first distillate composition comprising HF and entrainer and a first bottoms composition comprising E-HFC-1234ze and at least on of HFC-245fa or HFC-245eb; c) condensing said first distillate composition to form two liquid phases, being (i) an entrainer-rich phase and (ii) an HF-rich phase; and d) recycling the entrainer-rich phase back to the first distillation step.

The foregoing general description and the following detailed description are exemplary and explanatory only and are not restrictive of the invention, as defined in the appended claims.

### BRIEF DESCRIPTION OF THE DRAWINGS

Embodiments are illustrated in the accompanying figures to improve understanding of concepts as presented herein.
FIG. 1 is an illustration of one embodiment of an azeotropic distillation for the separation of HF and HFC-1234ze with no added entrainer.
FIG. 2 is an illustration of one embodiment of an azeotropic distillation for the separation of HF and HFC-1234ze with an added entrainer.
FIG. 3 is an illustration of one embodiment of a process to separate at least one of HFC-245fa or HFC-245eb from a mixture comprising HFC-1234ze, HF and said at least one of HFC-245fa or HFC-245eb via azeotropic distillation wherein HFC-1234ze acts as an entrainer followed by a process in which HFC-1234ze and HF are separated from a mixture comprising HFC-1234ze and HF, but now substantially free of HFC-245fa and/or HFC-245eb, by azeotropic distillation without the addition of another chemical compound to function as an entrainer.
FIG. 4 is an illustration of one embodiment of a process to separate HFC-1234ze and at least one of HFC-245fa or HFC-245eb from a mixture comprising HFC-1234ze, HF and said at least one of HFC-245fa or HFC-245eb via azeotropic distillation wherein a supplemental entrainer is fed to the distillation.
FIG 5 is an illustration of one embodiment of a process to separate at least one of HFC-245fa or HFC-245eb from a mixture comprising HFC-1234ze, HF and said at least one of HFC-245fa or HFC-245eb via azeotropic distillation wherein HFC-1234ze acts as an entrainer followed by a process in which HFC-1234ze and HF are separated from a mixture comprising HFC-1234ze and HF, but now substantially free of HFC-245fa and/or HFC-245eb, by azeotropic distillation with an added entrainer.
FIG 6 illustrates another embodiment of the process shown in Figure 3 wherein the two-phase mixture leaving the condenser of the first column is decanted and separated into HFC-1234ze-rich and HF-rich streams which are fed to the HFC-1234ze and HF columns, respectively,
FIG 7 illustrates another embodiment of the process shown in Figure 5 wherein the two-phase mixture leaving the condenser of the first column is decanted and separated into HFC-1234ze-rich and HF-rich streams which are fed to the HFC-1234ze and HF columns, respectively.
FIG 8 illustrates another embodiment of the process shown in FIG 6, wherein the three columns, 20, 110, and 220, share one decanter.

Skilled artisans appreciate that objects in the figures are illustrated for simplicity and clarity and have not necessarily been drawn to scale. For example, the dimensions of some of the objects in the figures may be exaggerated relative to other objects to help to improve understanding of embodiments.

### DETAILED DESCRIPTION

Many aspects and embodiments have been described above and are exemplary.

Other features and benefits of any one or more of the embodiments will be apparent from the following detailed description, and from the claims.

### 1. Definitions and Clarification of Terms

Before addressing details of embodiments described below, some terms are defined or clarified.

By azeotropic or azeotrope composition is meant a constant-boiling mixture of two or more substances that boils at a constant composition and thus behaves as a single substance. Constant-boiling compositions are characterized as azeotropic because they exhibit either a maximum or minimum boiling point, when compared with the boiling points of the individual components. Azeotropic compositions are also characterized by a minimum or a maximum in the vapor pressure measurements relative to the vapor pressure of the neat components in a PTx cell as a function of composition at a constant temperature. For homogenous azeotropes, where the vapor phase is in equilibrium with a single liquid phase, the compositions of the vapor and liquid phases are identical. However, for heterogeneous azeotropes, where the vapor phase is in equilibrium with two liquid phases, all three equilibrium phases can have different, but constant, compositions.

As used herein, the term "azeotrope-like composition" (also commonly referred to as a "near azeotropic composition") means a constant boiling, or substantially constant boiling liquid admixture of two or more substances that behaves as a single substance. One way to characterize an azeotrope-like composition is that the composition of the vapor produced by partial evaporation or distillation of the liquid does not change substantially throughout the partial evaporation or distillation. Similarly, the composition of the liquid phase or phases present does not change substantially during the partial evaporation or distillation. That is, the admixture boils/distills/refluxes without substantial composition change. This is to be contrasted with non-azeotrope-like compositions in which the liquid composition changes to a substantial degree during boiling or evaporation. Another way to characterize an azeotrope-like composition is that the bubble point vapor pressure of the composition and the dew point vapor pressure of the composition at a particular temperature are substantially the same. Herein, a composition is considered to be azeotrope-like if the difference in dew point pressure and bubble point pressure is less than or equal to 3 percent (based upon the bubble point pressure).

By high-boiling azeotrope is meant that an azeotropic or azeotrope-like composition boils at a higher temperature at any given pressure than any one of the compounds that comprise it would separately boil at that pressure. Alternately, by high-boiling azeotrope is meant any azeotropic or azeotrope-like composition that has a lower vapor pressure at any given temperature than any one of the compounds that comprise it would separately have at that temperature.

By low-boiling-azeotrope is meant that an azeotropic or azeotrope-like composition boils at a lower temperature at any given pressure than any one of the compounds that comprise it would separately boil at that pressure. Alternately, by low-boiling azeotrope is meant any azeotropic or azeotrope-like composition that has a higher vapor pressure at any given temperature than the vapor pressure of any one of the compounds that comprise the azeotrope would separately have at that temperature.

It is possible to characterize an azeotropic or azeotrope-like composition as a substantially constant-boiling admixture that may appear under many guises, depending upon the conditions chosen, by several criteria:
* The composition can be defined as an azeotrope of two compounds because the term "azeotrope" is at once both definitive and limitative, and requires effective amounts of those two or more compounds for this unique composition of matter which can be a constant-boiling composition.
* It is well known by those skilled in the art, that, at different pressures, the composition of a given azeotrope or azeotrope-like composition will vary at least to some degree, as will the boiling point temperature. Thus, an azeotropic or azeotrope-like composition of two compounds represents a unique type of relationship but with a variable composition which depends on temperature and/or pressure. Therefore, compositional ranges, rather than fixed compositions, are often used to define azeotropes and azeotrope-like compositions.
* An azeotrope or azeotrope-like composition of two compounds can be characterized by defining compositions characterised by a boiling point at a given pressure, thus giving identifying characteristics without unduly limiting the scope of the invention by a specific numerical composition, which is limited by and is only accurate as the analytical equipment available.

It is recognized in the art that both the boiling point and the weight (or mole) percentages of each component of the azeotropic composition may change when the azeotrope or azeotrope-like liquid composition is allowed to boil at different pressures. Thus, an azeotropic or an azeotrope-like composition may be defined in terms of the unique relationship that exists among components or in terms of the exact weight (or mole) percentages of each component of the composition characterized by a fixed boiling point at a specific pressure.

As used herein, the term "azeotrope" is meant to refer to azeotrope compositions and/or azeotrope-like compositions.

By entrainer is meant any compound that, when added to a first mixture, forms one or more azeotropes with the components of the mixture to facilitate separation of the components of the mixture. As used herein, the terms "entrainer" and "entraining agent" are used interchangeably and are to be interpreted as having identical meaning.

By azeotropic distillation is meant a process in which a distillation column is operated under conditions to cause one or more azeotropic or azeotrope-like composition to form, and thereby facilitates the separation of the components of the mixture. Azeotropic distillations may occur where only the components of the mixture to be separated are distilled, or where an entrainer is added that forms an azeotrope with one or more of the components of the initial mixture. Entrainers that act in this manner, that is to say, that form an azeotrope with one of more of the components of the mixture to be separated thus facilitating the separation of those components by distillation, are more commonly called azeotroping agents or azeotropic entrainers.

In conventional or azeotropic distillations, the overhead or distillate stream exiting the column may be condensed using conventional reflux condensers. At least a portion of this condensed stream can be returned to the top of the column as reflux, and the remainder recovered as product or for optional processing. The ratio of the condensed material which is returned to the top of the column as reflux to the material removed as distillate is commonly referred to as the reflux ratio. The compounds and entrainer exiting the column as distillate or distillation bottoms stream can then be passed to a stripper or second distillation column for separation by using conventional distillation, or may be separated by other methods, such as decantation. If desired, the entrainer may then be recycled back to the first distillation column for reuse.

The specific conditions which can be used for practicing the invention depend upon a number of parameters, such as the diameter of the distillation column, feed points, number of separation stages in the column, among others. In one embodiment, the operating pressure of the distillation system may range from about 5 to 500 psia (34.5 kPa to 3.5 MPa), in another embodiment, about 20 to 400 psia (138 kPa to 2.75 MPa). Normally, increasing the reflux ratio results in increased distillate stream purity, but generally the reflux ratio ranges between 1/1 to 200/1. The temperature of the condenser, which is located adjacent to the top of the column, is normally sufficient to substantially fully condense the distillate that is exiting from the top of the column, or is that temperature required to achieve the desired reflux ratio by partial condensation.

The problems associated with conventional distillation may be solved by a distillation process using an entrainer. The difficulty in applying this method is that there is no known way, short of experimentation, of predicting which if any compound will be an effective entrainer.

Hydrogen fluoride (HF, anhydrous) is a commercially available chemical or can be produced by methods known in the art.

As used herein, a fluoroolefin is a compound containing carbon, fluorine and optionally hydrogen and also at least one double bond. Fluoroolefins include but are not limited to 1,3,3,3-tetrafluoropropene (HFC-1234ze, CF₃CF=CH₂), 2,3,3,3-tetrafluoropropene (HFC-1234yf, CF₃CF=CH₂), 1,2,3,3,3-pentafluoropropene (HFC-1225ye, CF₃CH=CHF), and 3,3,3-trifluoropropene (HFC-1243zf, CF₃CH=CH₂), among others. Additionally, when referring to a fluoroolefin-rich phase, this may mean a single fluoroolefin or a mixture of two or more fluoroolefins.

1,3,3,3-tetrafluoropropene (HFC-1234ze, CF₃CF=CH₂) may be prepared by known methods, such as dehydrofluorination of 1,1,1,3,3-pentafluoropropane (HFC-245fa, CF₃CH₂CHF₂) or 1,1,2,3-pentafluoropropane (HFC-245eb, CF₃CHFCH₂F). HFC-245fa may be prepared by processes described in the art such as for instance in US Patent No. US 5,945,573 or US 6,376,727. HFC-245eb may be prepared by processes described in the art such as for instance in US Patent No. 5,396,000.

HFC-1214ze is a valuable fluorocarbon useful as a refrigerant, blowing agent, aerosol propellant, and sterilant among other uses. HFC-1234ze exists as either of two isomers, Z-HFC-1234ze and E-HFC-1234ze. Hereafter, by HFC-1234ze is meant either of the two isomers and/or mixtures of the two isomers.

HFC-1234ze may be prepared by the vapor phase dehydrofluorination of HFC-245fa or HFC-245eb by processes known in the art, such as those described in US Patent Nos, US 5,396,000, US 5,679,875, US 6,031,141, and US 6,389,284, For example, HFC-1234ze can be prepared by passing HFC-245fa, HFC-245eb or mixtures of HFC-245fa and HFC-245eb over a chrome oxide catalyst at elevated temperatures, for example, at above 300 °C. The product stream from this reacti on contains HFC-1234ze, HF and any unreacted HFC-245fa and/or HFC-245eb.

U.S. Patent Publication no. 2007-0100173 A1 discloses the azeotrope and azeotrope-like (also known as near-azeotrope) compositions for *E*-HFC-1234ze and HF. These azeotrope and azeotrope-like compositions may be used in processes for separating an HFC-1234ze from a mixture comprising HF and HFC-1234ze. Additionally, as HFC-1234ze may be prepared by dehydrofluorination of HFC-245fa or HFC-245eb the compositions as described therein may be used in similar methods for separation or purification of HFC-1234ze from mixtures comprising HFC-1234ze, HF and at least one of HFC-245fa or HFC-245eb.

The term "entrainer" is used herein to describe any compound that would be effective in separation of fluoroolefins from mixtures comprising HF and fluoroolefin in an azeotropic distillation process. Included as useful entrainers are those compounds that form azeotropes with one or more of the components of a mixture, including fluoroolefins, HF, and possible hydrofluarocarbons for which the boiling point of at least one of such azeotropes is lower than the boiling point of the fluoroolefin/HF azeotrope.

Entrainers may be selected from the group consisting of hydrocarbons, chlorocarbons, chlorofluorocarbons, hydrochlorofluorocarbons, hydrofluorocarbons, perfluorocarbons, fluoroethers, HFPO, SF₆, chlorine, hexafluoroacetone, and mixtures thereof.

Hydrocarbon entrainers comprise compounds containing 1 to 5 carbon atoms and hydrogen, Hydrocarbon entrainers may be linear, branched, cyclic, saturated or unsaturated compounds. Representative hydrocarbon entrainers include but are not limited to methane, ethane, ethylene, acetylene, vinylacetylene, n-propane, propylene, propyne, cyclopropane, cyclopropene, propadiene, n-butane, isobutane, 1-butene, isobutene, 1,3-butadiene, 2,2-dimethylpropane, cis-2-butene, trans-2-butene, 1-butyne, n-pentane, isopentane, neopentane, cyclopentane, 1-pentene, 2-pentene, and mixtures thereof.

Chlorocarbon entrainers comprise compounds containing carbon, chlorine and optionally hydrogen, including but not limited to methylene chloride (CH₂Cl₂), and methyl chloride (CH₃Cl).

Chlorofluorocarbon (CFC) entrainers comprise compounds with carbon, chlorine and fluorine. Representative CFCs include but are not limited to dichlorodifluoromethane (CFC-12), 2-chloro-1,1,2-trifluoroethylene, chloropentaftuoroethane (CFC-115), 1,2-dichloro-1,1,2,2-tetrafluotoethane (CFC-114), 1,1-dichloro-1,2,2,2-tetrafluoroethane (CFC-114a), 1,1,2-trichloro-1,2,2-trifluoroethane (CFC-113), 1,1,1-trichloro-2,2,2-trifluoroethane (CFC-113a), 1,1,2-trichloro-1,2,3,3,3-pentafluoropropane (CFC-215bb), 2,2-dichloro-1,1,1,3,3,3-hexafluoropropane (CFC-216aa), 1,2-dichloro-1,1,2,3,3,3-hexafluoropropane (CFC-216ba), 2-chloro-1,1,1,2,3,3,3-heptafluoropropane (CFC-217ba), 2-chloro-1,1,3,3,3-pentafluoropropene (CFC-1215xc), and mixtures thereof.

Hydrochlorofluorocarbon (HCFC) entrainers comprise compounds with carbon, chlorine, fluorine and hydrogen. Representative HCFCs include but are not limited to dichlorofluoromethane (HCFC-21), 1,1-dichloro-3,3,3-trifluoroethane (HCFC-123), 1,1-dichloro-1-fluoroethane (HCFC-141b), 2-chloro-1,1,1,2-tetrafluoroethane (HCFC-124), 1-chloro-1,1,2,2-tetrafluoroethane (HCFC-124a), 2-chloro-1,1,1-trifluoroethane (HCFC-133a), 1-chloro-1,1-difluoroethane (HCFC-142b), 2-chloro-1,1-difluoroethylene (HCFC-1122), and mixtures thereof.

Hydrofluorocarbon (HFC) entrainers comprises compounds that contain carbon, hydrogen and fluorine. Representative HFCs include but are not limited to 1,1,2-trifluoroethylene (HFC-1123), 1,1-difluoroethylene (HFC-1132a), 1,2,3,3,3-pentafuoropropene (HFC-1225ye, either of the Z-or E-isomers or a mixture thereof), 1,1,3,3,3-pentafluoropropene (HFC-1225zc), 2,3,3,3-tetrafluoropropene (HFC-1234yf), 3,3,3-trifluoropropene (HFC-1243zf), 1,3,3,3-tetrafiuoropropene (HFC-1234ze, either of the Z- or E- isomers or a mixture thereof), and mixtures thereof.

Perfluorocarbon (PFC) entrainers comprise compounds with carbon and fluorine only. Representative PFCs include but are not limited to hexafluoroethane (PFC-116), octafluoropropane (PFC-218), 1,1,1,4,4,4-hexafluoro-2-butyne (PFBY-2), hexafluoropropylene (HFP, PFC-1216), hexafluorocyclopropane (PFC-C216), octafluorocyclobutane (PFC-C316), decafluorobutane (PFC-31-10, any isomer(s)), 2,3-dichloro-1,1,1,4,4,4-hexafluoro-2-butene (PFC-1316mxx), octafluoro-2-butene (PFC-1318my, cis and trans), hexafluorobutadiene (PFC-2316), and mixtures thereof.

Fluoroether entrainers comprise compounds with carbon, fluorine, optionally hydrogen and at least one ether group oxygen. Representative fluoroethers include trifluoromethyl-difluoromethyl ether (CF₃OCHF₂, HFOC-125E), 1,1-difluorodimethyl ether, tetrafluorodimethylether (HFOC-134E), difluoromethyl methyl ether (CHF₂OCH₃, HFOC-152aE), pentafluoroethyl methyl ether, and mixtures thereof.

Miscellaneous other compounds that may be useful as entrainers include HFPO, chlorine (Cl₂), hexafluoroacetone, PMVE (perfluoromethylvinylether), PEVE (perfluoroethylvinylether), and mixtures thereof.

Entrainers as described above are available commercially or may be produced by methods known in the art.

As used herein, by "essentially free of" is meant that a composition contains less than 100 ppm (mole basis), less than 10 ppm or less than 1 ppm, of the specified component. If a composition is essentially free of more than one component, then the total concentration of those components is less than 100 ppm, less than 10 ppm, or less than 1 ppm.

The process equipment for all the processes disclosed herein and the associated feed lines, effluent lines and associated units may be constructed of materials resistant to hydrogen fluoride. Typical materials of construction, well-known to the art, include stainless steels, in particular of the austenitic type, and the well-known high nickel alloys such as Monel^{®} nickel-copper alloys, Hastelloy^{®} nickel based alloys and Inconel^{®} nickel-chromium alloys,

As used herein, the terms "comprises," "comprising," "includes," "including," "has," "having" or any other variation thereof, are intended to cover a non-exclusive inclusion. For example, a process, method, article, or apparatus that comprises a list of elements is not necessarily limited to only those elements but may include other elements not expressly listed or inherent to such process, method, article, or apparatus. Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

Also, use of "a" or "an" are employed to describe elements and components described herein, This is done merely for convenience and to give a general sense of the scope of the invention. This description should be read to include one or at least one and the singular also includes the plural unless it is obvious that it is meant otherwise.

Group numbers corresponding to columns within the Periodic Table of the elements use the "New Notation" convention as seen in the CRC Haddbook of Chemistry and Physics, 81st Edition (2000-2001).

Unless otherwise defined, all technical and scientific terms used herein have the same meaning as commonly understood by one of ordinary skill in the art to which this invention belongs. Although methods and materials similar or equivalent to those described herein can be used in the practice or testing of embodiments of the present invention, suitable methods and materials are described below.

### 2. Separation process - Azeotropic distillation with no entrainer

It has been discovered that some fluoroolefins form azeotrope compositions with HF. Generally, the fluoroolefin/HF azeotrope composition will boil at a lower temperature than either of the corresponding pure compounds. Several examples of such fluoroolefin/HF azeotropes are disclosed in U.S. Patent Publication numbers 2007-0100173 A1, 2007-0100174 A1, 2007-0099811 A1, 2007-0100175 A1, 2007-0100176 A1, and 2006-0116538 A1.

It has been unexpectedly calculated that in a few cases azeotrope compositions comprising fluoroolefins and HF may form two liquid phases when condensed and/or cooled, The two phases comprise a fluoroolefinrich phase and an HF-rich phase. This phase behavior allows unique separation schemes utilizing liquid-liquid separation (such as decantation) of the two phases that are not possible with many saturated hydrofluorocarbons, which in general do not phase separate in the same manner.

In one embodiment, the present disclosure provides a process for separating a mixture comprising HF and HFC-1234ze, said process comprising a) feeding the composition comprising HF and HFC-1234ze to a first distillation column; b) removing an azeotrope composition comprising HF and HFC-1234ze as a first distillate and either i) HF or ii) HFC-1234ze as a first column bottoms composition; c) condensing the first distillate to form two liquid phases, being i) an HF-rich phase and ii) an HFC-1234ze-rich phase; and d) recycling a first liquid phase enriched in the same compound that is removed as the first column bottoms, said first liquid phase being either i) HF-rich phase or ii) HFC-1234ze-rich phase, back to the first distillation column.

Additionally, in another embodiment, the process as described in the paragraph above may further comprise feeding a second liquid phase not recycled in step (d), said second liquid phase being either i) HF-rich phase or ii) HFC-1234ze-rich phase, to a second distillation zone, and recovering the compound not recovered in step (b) as the first column bottoms composition as the second column bottoms composition.

In another embodiment, a process is provided for separating HFC-1234ze from a mixture comprising hydrogen fluoride and said HFC-1234ze, wherein said HFC-1234ze is present in a concentration greater than the azeotrope concentration for hydrogen fluoride and said HFC-1234ze, said process comprising: a) feeding said mixture comprising hydrogen fluoride and said HFC-1234ze to a first distillation column; b) removing an azeotrope composition comprising hydrogen fluoride and HFC-1234ze as a first distillate from the first distillation column; c) recovering HFC-1234ze essentially free of hydrogen fluoride as a first bottoms composition from the first distillation column; and d) condensing the first distillate to form two liquid phases, being i) a hydrogen fluoride-rich phase and ii) an HFC-1234ze-rich phase; and e) recycling the HFC-1234ze-rich phase to the first distillation column,

In another embodiment, the process may further comprise: a) feeding the hydrogen fluoride-rich phase to a second distillation column, and b) recovering hydrogen fluoride essentially free of HFC-1234ze from the bottom of the second distillation column.

In another embodiment, the second distillate comprising HF and HFC-1234ze may be recycled to the two liquid phases.

In one embodiment, wherein the composition comprising HF and HFC-1234ze has a concentration of HFC-1234ze that is greater than the azeotrope concentration for HFC-1234ze and HF, the first distillation column removes the excess HFC-1234ze from the bottom of the column and the azeotrope composition exits the top of the column as the distillate. In another embodiment, the azeotrope composition compnsing HF and HFC-1234ze may be condensed and cooled thereby forming two liquid phases, an HF-rirh phase and an HFC-1234ze-rich phase,

In one embodiment, the HFC-1234ze-rich phase is recycled back to the first distillation column and the HF-rich phase is fed to a second distillation column. As the HF-rich phase may have HF in excess of the azeotrope composition for HF/HFC-1234ze, the excess HF will be removed from the second distillation column bottom.

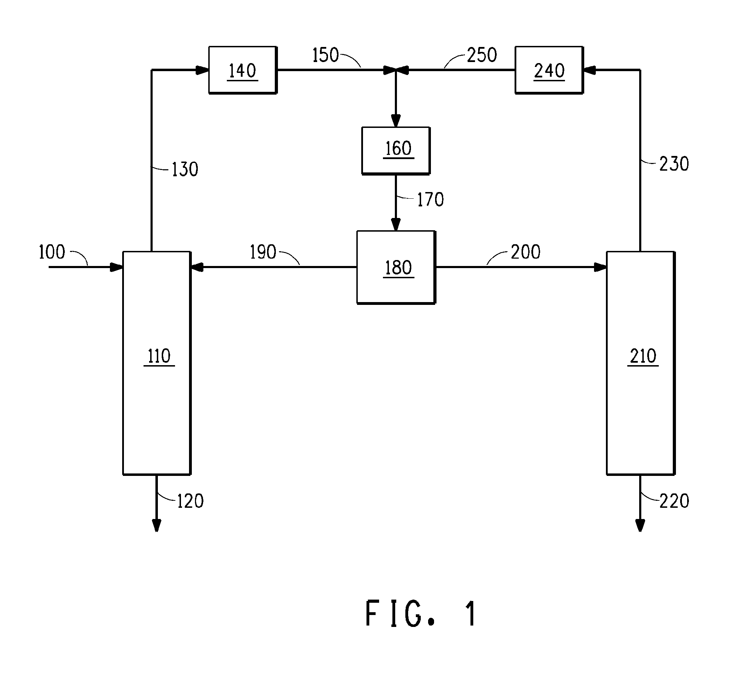

Referring now to FIG 1, one embodiment of this process is illustrated. A composition comprising HF and HFC-1234ze is fed to a first column 110 via stream 100. This first column is operated under appropriate conditions to approach the low-boiling HF/HFC-1234ze azeotrope. Because HF is being fed to this first column in excess of that needed to form the azeotrope with the HF, HFC-1234ze is recovered as the bottoms of the column via stream 120, while a composition near to the HF/HFC-1234ze azeotrope is recovered as distillate via stream 130. Stream 130 is condensed in 140, mixed with a nearly azeotropic composition receded from a second column 210 via stream 250 and the combined stream is sub-cooled in cooler 160 and sent to decanter 180 where the combined stream 170 separates into a separate HFC-1234ze-rich stream 190 and an HF-rich stream 200. Stream 190 is recycled to the first column as reflux. Stream 200 is fed to the top stage of the second distillation column 210, operated under conditions to approach the HF/HFC-1234ze azeotrope. Because the HF is being fed to this second column in excess of that needed to form the low-boiling HF/HFC-1234ze azeotrope, HF is recovered as the bottoms of the column via stream 220 while a composition close to the HF/HFC-1234ze azeotrope is recovered as distillate via stream 230. Stream 230 is condensed in 240, mixed with the nearly azeotropic composition from the first column via stream 150 and fed to cooler 160 and then decanter 180.

In another embodiment, a process is provided for separating hydrogen fluoride from a mixture comprising hydrogen fluoride and an HFC-1234ze, wherein hydrogen fluoride is present in a concentration greater than the azeotrope concentration for hydrogen fluoride and said HFC-1234ze, said process comprising: a) feeding said mixture comprising hydrogen fluoride and HFC-1234ze to a first distillation column; b) removing an azeotrope composition comprising HFC-1234ze and HF as a first distillate from the first distillation column; c) recovering hydrogen fluoride essentially free of HFC-1234ze from the bottom of the first distillation column; d) condensing the first distillate to form two liquid phases, being an HFC-1234ze-rich phase and a hydrogen fluoride-rich phase; and e) recycling the HF-rich phase to the first distillation column.

In another embodiment, the process may further comprise: a) feeding the HFC-1234ze-rich phase to a second distillation column; and b) recovering HFC-1234ze essentially free of hydrogen fluoride from the bottom of the second distillation column.

In another embodiment, the process may further comprise: recycling the hydrogen fluoride-rich phase to the first distillation column,

In another embodiment, the composition cornprising HF and HFC-1234ze has a greater concentration of HF than the azeotrope composition for HF and HFC-1234ze. The excess HF may be removed from the bottom of the first distillation column and the azeotrope composition exits as the distillate. In another embodiment, the azeotrope composition comprising HF and HFC-1234ze may be condensed and cooled thereby forming two liquid phases, an HF-rich phase and an HFC-1234ze-rich phase. For this embodiment, the HF-rich phase is recycled back to the first distillation column and the HFC-1234ze-rich phase is fed to a second distillation column. As the HFC-1234ze-rich phase may have HFC-1234ze in excess of the azeotrope composition for HF/HFC-1234ze, the excess HFC-1234ze may be removed from the second distillation column bottom as HFC-1 234ze essentially free of HF.

Referring again to FIG 1, another embodiment of this process is illustrated. A composition comprising HF and HFC-1234ze is fed to a first column 110 via stream 100. This first column is operated under appropriate conditions to approach the low-boiling HF/HFC-1234ze azeotrope. Because HF is being fed to this first column in excess of that needed to form the azeotrope with HFC-1234ze, HF is recovered as a product stream from the bottom of the column via stream 120, while a composition near to the HF/HFC-1 234ze azeotrope is recovered as distillate via stream 130, Stream 130 is condensed in condenser140, mixed with a nearly azeotropic composition recycled from a second column via stream 250 and the combined stream is sub-cooled in cooler 160 and sent to decanter 180 where the combined stream 170 separates into a separate HF-rich stream 190 and HFC-1234ze-rich stream 200. Stream 190 is recycled to the first column as reflux. Stream 200 is fed to the top stage of the second distillation column 210, operated under conditions to approach the HF/HFC-1234ze azeotrope, Because HFC-1234ze is being fed to this second column in excess of that needed to form the low-boiling HF/HFC-1234ze azeotrope, HFC-1234ze is recovered from the bottom of the column via stream 220, while a composition close to the HF/HFC-1234ze azeotrope is recovered as distillate via stream 230. Stream 230 is condensed in condenser 240, mixed with the nearly azeotropic composition from the first column via stream 150 and fed to cooler 160 and then decanter 180.

In one embodiment, the operating conditions for the first and second distillation columns will depend upon the HFC-1234ze being purified and the relative amounts of HF and HFC-1234ze in the composition to be separated.

In one embodiment, the first and second distillation column may operate at from about 14.7 psia (101 kPa) to about 300 psia (2068 kPa), with a top temperature of from about -50°C to about 200 °C and a bottom temperature from about -30 °C to about 220°C. In another embodiment, the pressure will range from about 50 psia (345 kPa) to about 250 psia (1724 kPa), with a top temperature of from about -25 °C to about 100 °C and a bottom temperature from about 0 °C to about 150 °C,

### 3. Separation process - Azeotropic distillation with an entrainer

Azeotropic distillation for separating HFC-1234ze from mixtures of Hand HFC-1234ze may, in another embodiment, be carried out using an entrainer compound. For the process including an entrainer, the azeotrope composition need not phase separate upon condensing and cooling as described above.

In one embodiment, the entrainer serves to provide an improved liquid-liquid phase separation for a system wherein that separation would otherwise not be effective.

In one embodiment, the HFC-1234ze is present in the HF/HFC-1234ze mixture in a concentration greater than the azeotrope concentration for said HFC-1234ze and HF. Thus, in one embodiment is provided a process for the purification of an HFC-1234ze from a mixture comprising HFC-1234ze and HF, wherein said HFC-1234ze is present in said mixture in a concentration greater than the azeotrope concentration for said HFC-1234ze and HF, said process comprising:
a. adding an entrainer to the mixture comprising HFC-1234ze and HF thus forming a second mixture:
b. distilling said second mixture in a first distillation step to form a first distillate composition comprising HF, HFC-1234ze, and entrainer, and a first bottoms composition comprising HFC-1234ze essentially free of HF and entrainer;
c. condensing said first distillate composition to form two liquid phases, being i) an HF-rich phase and ii) an entrainer-rich phase; and
d. optionally recycling the entrainer-rich phase back to the first distillation step.

In another embodiment, the process further comprises feeding the HF-rich phase to a second distillation step and forming a second distillate composition comprising entrainer, HFC-1234ze and HF and a bottoms composition comprising HF essentially free of HFC-1234ze and entrainer, In another embodiment, the process may further comprise recycling said second distillate composition back to the two liquid phases.

The process for separating an HFC-1234ze from a first composition comprising HF and HFC-1234ze comprises contacting said first composition with an entrainer to form a second composition. The contacting may occur in a first distillation column, or the second composition may be formed by mixing the components prior to feeding to a distillation column in a pre-mixing step.

The weight ratio of the HF and HFC-1234ze in the first composition will depend upon the means of producing the composition. In one embodiment, the HF may be from about 3 weight percent to about 85 weight percent of the composition; the HFC-1234ze may be from about 97 weight percent to about 15 weight percent.

In another embodiment, the HF may be from about 5 weight percent to about 50 weight percent and the HFC-1234ze may be from about 95 weight percent to about 50 weight percent

In yet another embodiment the composition comprising HF and HFC-1234ze may be produced in a dehydrofluorination reactor resulting in a 50/50 mole ratio of HF to the HFC-1234ze.

In one embodiment, the compositions comprising HF and HFC-1234ze may be prepared by any convenient method to combine the desired amounts of the individual components. A preferred method is to weigh the desired component amounts and thereafter combine the components in an appropriate vessel. Agitation may be used, if desired.

Alternatively, the compositions comprising HF and HFG-1234ze may be prepared by feeding the effluent from a reactor, including a dehydrofluorination reactor that contains HF and HFC-1232ze, to the first distillation column. The entrainer may be added at a separate feed point such that the second composition is formed directly in the distillation column. Alternatively, the entrainer may be mixed with the first composition comprising HF and HFC-1234ze thus forming the second composition prior to the distillation column in a pre-mixing step.

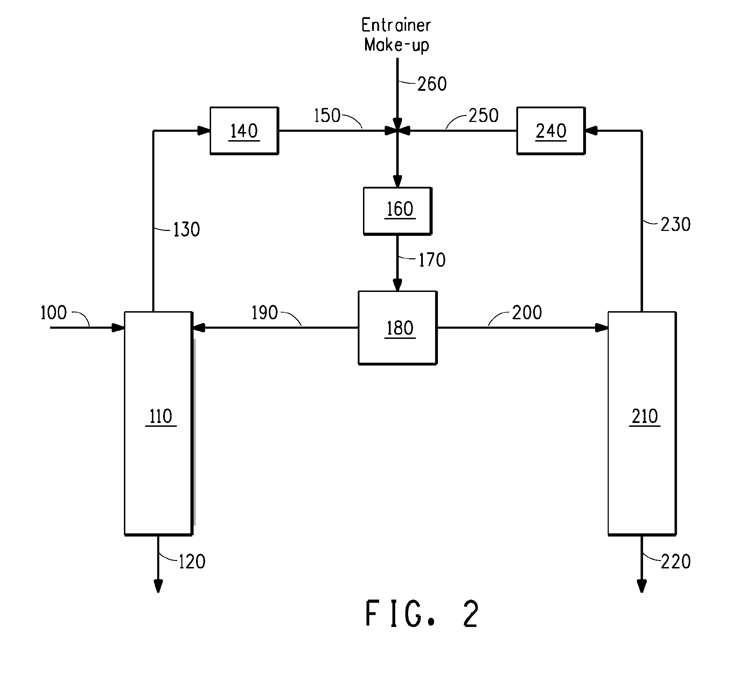

In one embodiment of the separation process, a composition comprising HFC-1234ze and HF is fed directly to a first distillation column. In another embodiment, the HFC-1234ze and HF may be pre-mixed with an entrainer prior to the distillation column. The pre-mixing step may occur in a cooler (160 in FIG 2). Then the cooled mixture is fed to a decanter (180 in FIG 2) prior to feeding to the distillation column.

In one embodiment, the first distillate composition comprises a low boiling azeotrope of HF and entrainer optionally containing minor amounts of HFC-1234ze. Further, in another embodiment the HFC-1234ze essentially free of HF and optionally minor amounts of entrainer may be recovered from the bottom of the first distillation column.

The operating variables for the first distillation column will depend strongly on the entrainer being used in the separation process. In general the first distillation column may operate at pressures from about 14.7 psia (101 kPa) to about 500 psia (3448 kPa) with a top temperature of from about -50 °C to about 100 °C and a bottom temperature of from about -30 °C to about 200°C. In another embodiment, the first distillation column will operate at pressures from about 100 psia (690 kPa) to about 400 psia (2758 kPa) with a top temperature of from about - 50 °C to about 50 °C and a bottom temperature from about 10 °C to about 150 °C.

It was surprisingly calculated that in some few cases, azeotropes of HF and compounds used as entrainers will separate into HF-rich and entrainer-rich liquid fractions upon condensing and being cooled. In one embodiment, the first distillate composition may be fed to a liquid separation zone (e.g. decanter). The first distillate composition comprising an azeotrope of HF and entrainer may be phase separated forming two liquid phases, one being HF-rich and the other being entrainer-rich. The lower density phase may be recovered from the top of the liquid separation zone and the higher density phase may be recovered from the bottom of the liquid separation zone. The entrainer-rich phase (whether higher or lower density) may be fed back to the first distillation column. In one embodiment the HF-rich phase may be fed to a second distillation column or in another embodiment, the HF-rich phase may be split to send some portion back to the first distillation column (in order to provide more reflux and allow the first distillation column to operate property) and the remainder may be fed to the second distillation column. The second distillation column allows recovery of HF essentially free of HFC-1234ze and entrainer as a bottoms composition. The top composition comprising HFC-1234ze, HF and entrainer may be recycled to the liquid separation zone, be utilized in some other manner, or disposed. The operating variables for the second distillation column will depend strongly on the entrainer being used in the separation process.

In general the second distillation column may operate at pressures from about 14.7 psia (101 kPa) to about 500 psia (3448 kPa) with a top temperature of from about -50 °C to about 100 °C and a bottom temperature of from about 30 °C to about 200 °C. In another embodiment, the first distillation column will operate at pressures from about 100 psia (690 kPa) to about 400 psia (2758 kPa) with a top temperature of from about -25 °C to about 50 °C and a bottom temperature from about zero °C to about 150 °C,

Referring now to FIG 2, a composition comprising HF and HFC-1234ze is fed to a first distillation column 110 via stream 100. An entrainer-rich composition is also fed to the top stage of column 110 via stream 190. If the combined amount of HFC-1234ze in streams 100 and 190 is in excess of that needed to form the low-boiling HF/HFC-1234ze azeotrope, HFC-1234ze is recovered essentially free of both HF and entrainer from the bottom of column 110 via stream 120. A ternary composition comprising HF, HFC-1234ze, and entrainer, but enriched in HFC-1234ze relative to stream 190, leaves the top of the first column as the first distillate stream 130. Stream 130 is condensed by condenser 140 forming stream 150 and mixed with a condensed second distillate stream 250 from a second distillation column. In one embodiment, additional entrainer may be added via stream 260, if needed. Combined streams 150, 250, and 260 are fed to cooler 160 and then to decanter 180 where the sub-cooled liquid stream 170 separates into entrainer-rich and HF-rich liquid phase compositions which leave the decanter via streams 190 and 200, respectively. The HFC-1234ze present distributes between the two liquid phases with the majority ending up in the entrainer-rich phase, The HF-rich composition stream 200 is fed to the top stage of the second distillation column 210. Because the amount of HF in stream 200 is in excess of that needed to form a low-boiling HF/HFC-1234ze azeotrope, HF is recovered as a product stream essentially free of both HFC-1234ze and entrainer from the bottom of column 210 via stream 220. A ternary composition comprising HF, HFC-1234ze and entrainers, but enriched in entrainer relative to stream 200, leaves the top of the second column as the second distillate stream 230. Stream 230 is condensed in condenser 240, forming stream 250, and combined with streams 150 and 260 previously described.

Alternatively, in another embodiment, rather than feed the HF/HFC-1234ze mixture directly to the distillation column 110, the mixture may be fed to cooler 160 and then to decanter 180 where the mixture phase separates. Then stream 190 carries the mixture of HF, HFC-1234ze and entrainer to the first distillation column 110.

In another embodiment, the concentration of HF in the HF/HFC-1234ze mixture is greater than the concentration in the azeotrope of HFC-1234ze and HF. Thus, in another embodiment is provided a process for the purification of HF from a mixture comprising an HFC-1234ze and HF. wherein HF is present in a concentration greater than the azeotrope concentration for HF and said HFC-1234ze, said process comprising:
a. adding an entrainer to the mixture comprising HFC-1234ze and HF thus forming a second mixture;
b. distilling said second mixture in a first distillation step to form a first distillate composition comprising HF, entrainer, and an HFC-1234ze, and a first bottoms composition comprising HF essentially free of HFC-1234ze and entrainer;
c. condensing said first distillate composition to form two liquid phases, being i) an entrainer-rich phase and ii) an HF-rich phase; and
d. optionally recycling the HF-rich phase back to the first distillation step. In another embodiment, the process may further comprising feeding the HF-rich phase to a second distillation step and forming a second distillate composition comprising entrainer, HF, and HFC-1234xe, and a bottoms composition comprising HFC-1234ze essentially free of entrainer. In another embodiment, the process may further comprise recycling said second distillate composition back to the two liquid phases.

Referring again to FIG 2, a composition comprising HF and HFC-1234ze is fed to a first distillation column 110 via stream 100. An HF-rich composition is also fed to the top stage of column 110 via stream 190. If the combined amount of HF in streams 100 and 190 is in excess of that needed to form the low-boiling HF/HFC-1234ze and HF/entrainer azeotropes, HF is recovered essentially free of both HFC-1234ze and entrainer from the bottom of column 110 via stream 120. A composition enriched in HFC-1234ze and entrainer is recovered as the first distillate via stream 130. Stream 130 is condensed by condenser 140 forming stream 150 and mixed with a condensed second distillate stream 250 from a second distillation column. In one embodiment, additional entrainer may be added via stream 260, if needed. Combined streams 150, 250, and 260 are fed to cooler 160 and then to decanter 180 where the sub-cooled liquid stream 170 separates into HF-rich and entrainer-rich liquid phase compositions which leave the decanter via streams 190 and 200, respectively. The HFC-1234ze present distributes between the two liquid phases with the majority ending up in the entrainer-rich phase. The entrainer-rich composition stream 200 is fed to the top stage of the second distillation column 210. Because the amount of HFC-1234ze in stream 200 is in excess of that needed to form a low-boiling entrainer/HFC-1234ze azeotrope, HFC-1234ze is recovered as a product stream essentially free of both HF and entrainer from the bottom of column 210 via stream 220. A ternary composition comprising entrainer, HFC-1234ze, and HF, but enriched in entrainer relative to stream 200 leaves the top of the second column as the second distillate stream 230. Stream 230 is condensed in condenser 240, forming stream 250, and combined with streams 150 and 260 previously described.

Alternatively, in another embodiment, rather than feed the HF/HFC-1234ze mixture directly to the distillation column 110, the mixture may be fed to cooler 160 and then to decanter 180 where the mixture phase separates. Then stream 190 carries the mixture of HF, HFC-1234ze and entrainer as the HF-rich phase to the first distillation column 110.

### 4. Separation of HFC-245fa and/or HFC-245eb from HFC-1234ze and HF

HFC-1234ze may be produced by dehydrofluorination of certain HFC-245 (pentafluoropropane) isomers. By HFC-245 is meant any isomer of pentafluoropropane and any combinations of any isomers of pentafitioropropane that can yield HFC-1234ze upon dehydrofluorination. Isomers of hexafluoropropane include HFC-245fa (1,1,1,2,3,3-hexafluoropropane) and HFC-245eb (1,1,1,2,2,3-hexafluoropropane).

HFC-1234ze may be prepared by the vapor phase dehydrofluorination of HFC-245fa or HFC-245eb by processes known in the art, such as those described in US Patent Nos. US 5,895,825, US 5,986,151, US 6,031,141 and US 6,548,719, and also by methods disclosed in WO 2004/018093, WO 2004/018095, and JP 1999/140002. For example, HFC-1234ze can be prepared by passing HFC-245fa, HFC-245eb or mixtures of HFC-245fa and HFC-245eb over a chrome oxide catalyst at elevated temperatures, for example, at above 300 °C. The product stream from this reaction contains HFC-1234ze, HF and any unreacted HFC-245fa and/or HFC-245eb.

In one embodiment, a process is provided for the separation of HFC-1234ze from a mixture of HFC-1234ze, HF, and at least one of HFC-245fa or HFC-245eb, said process comprising:
a) subjecting said mixture to a first distillation step, wherein additional HFC-1234ze is fed from a second distillation step, to form a first distillate comprising an azeotrope of HFC-1234ze and HF and a first bottoms composition comprising at least one of HFC-245fa or HFC-245eb;
b) feeding said first distillate to a second distillation step to form a second distillate comprising an azeotrope of HFC-1234ze and HF and a second bottoms composition comprising HFC-1234ze essentially free of HF;
c) condensing said second distillate to form two liquid phases, being i) an HF-rich phase and ii) an HFG-1234ze-rich phase; and
d) recycling the HFC-1234ze-rich phase from (c) back to the second distillation step. In another embodiment, the process may further comprise feeding the HF-rich phase to a third distillation step to form a third distillate comprising an azeotrope of HFC-1 234ze and HF and a third bottoms composition comprising HF essentially free of HFC-1234ze.

In this embodiment the azeotropic distillation involves providing an excess of HFC-1234ze to the distillation column in addition to that produced from the dehydrofluorination reaction of HFC-245fa and/or HFC-245eb. In this embodiments, HFC-1234ze serves as an entrainer in the distillation process. If the proper total amount of HFC-1234ze is fed to the column, then all the HF may be taken overhead as an azeotrope composition containing HFC-1234ze and HF. Enough HFC-1234ze can be provided, for example, by feeding supplemental HFC-1234ze to the distillation column over that exiting in the dehydrofluorination reaction product stream. Thus, the HFC-245fa and/or HFC-245eb removed from the column bottoms may be essentially free of HF.

For example, a reactor product mixture comprising HF, HFC-1234ze and HFC-245fa may be fed to a first distillation column operated under conditions to form the HF/ HFC-1234ze azeotrope with the HF/ HFC-1234ze azeotrope being removed from the distillation column as the overhead distillate, The HF in this distillate may then be separated and removed from the HFC-1234ze by other means, e.g. by using pressure swing distillation or the methods as disclosed herein. Some portion of the HFC-1234ze so obtained can be recycled back to the first distillation column in quantities sufficient so that all the HF fed to the first distillation column is removed from that column as the HF/ HFC-1234ze azeotrope, thus producing a HFC-245fa bottoms stream essentially free of HF.

Where the composition to be separated is formed by dehydrohalogenating either of HFC-245fa or HFC-245eb, it is desirable to recycle any unreacted HFC-245fa or HFC-245eb back to the reactor so that they may be converted to HFC-1234ze. However, it is necessary that HF and HFC-1234ze be removed from said unreacted HFC-245fa or HFC-245eb prior to being recycled so as not to inhibit the equilibrium reaction, It is also necessary that the HF be removed from the HFC-1234ze to allow its use as a refrigerant or in other applications.

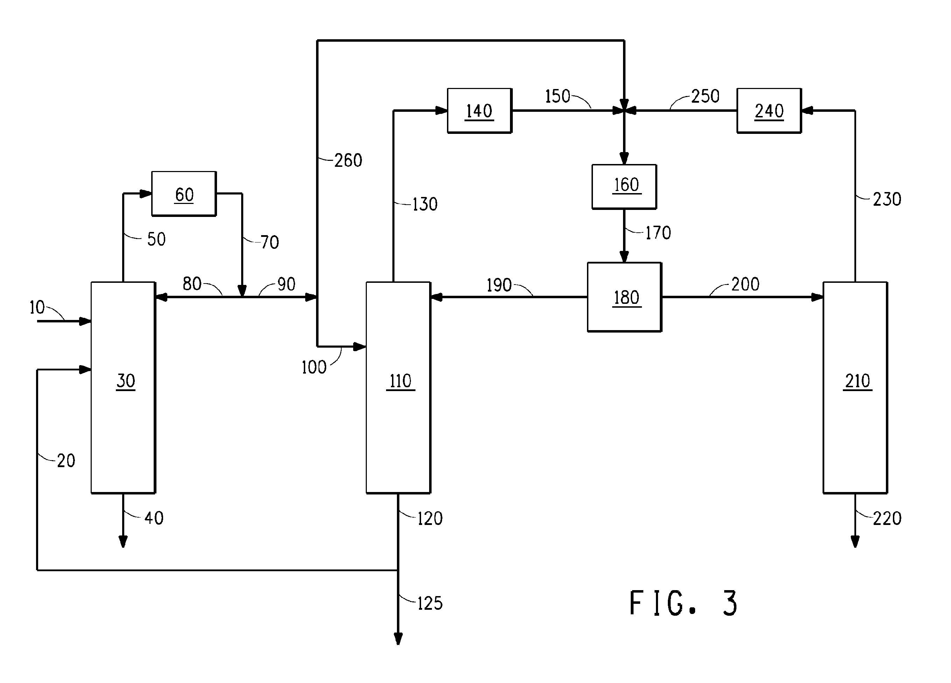

Referring now to FIG 3, a stream comprising HF, HFC-1234ze, and at least one of HFC-245fa or HFC-245eb is fed to a first distillation column via stream 10, with the column operated under conditions to approach the low-boiling HF/HFC-1234ze azeotrope, which is removed via streams 50, 70, and 90. Enough supplemental HFC-1234ze is recycled from the second column bottoms to this first column via stream 20 to enable all of the HF to be removed from the HFC-245eb and/or HFC-245fa. The HFC-245eb and/or HFC-245fa are obtained essentially, free of HFC-1234ze and HF as the bottoms product from this column via stream 40,

The near HF/HFC-1234ze azeotropic composition in stream 50 is condensed in condenser 60 and the resulting stream 70 is divided into reflux 80 and distillate 90 streams. Distillate stream 90 may be fed to a second distillation column 110 via stream 100 as shown and indicated, mixed with distillate streams 150 and 250 from the second and third columns, respectively, and sent to cooler 160 and decanter 180, or stream 90 may be divided between these two destinations. Because of the desire to remove all of the HF overhead in column 30, excess HFC-1234ze would be recycled to column 30, making the composition of streams 50, 70, 80, 90, and 100 lie on the HFC-1234ze-rich side of the azeotrope. Therefore, if distillate stream 90 is sent via stream 100 to a second distillation column, it should be sent to the column which produces purified HFC-1234ze as the bottoms product.

In one embodiment, distillate stream 90 via stream 260 is mixed with distillate streams 150 and 250 from the second and third columns, respectively, and sent to cooler 160, forming sub-cooled stream 170, which is fed to decanter 180. In the decanter, stream 170 separates into HFC-1234ze-rich and HF-rich liquid fractions, which are removed as streams 190 and 200. The HFC-1234ze-rich stream from the decanter is fed via stream 190 to a second distillation column 110 containing 19 theoretical stages and operated under conditions to approach the HFC-1234ze/HF azeotrope, which is distilled overhead as distillate stream 130, condensed in condenser 140, and mixed with the distillates from the first and third columns via stream 150. Column 110 produces a bottoms stream of HFC-1234ze essentially free of HF via stream 120. Part of the HFC-1234ze bottoms stream 120 is recycled to the first column via stream 20, as previously described, and the rest becomes the purified HFC-1234ze product removed via stream 125. The HF-rich stream from the decanter is fed via stream 200 to a third distillation column 210 operated under conditions to approach the HFC-1234xe/HF azeotrope, which is distillled overhead as distillate as stream 230 which is condensed in condenser 240 and mixed with the distillates from the first and second columns via stream 250, Column to produces a bottoms stream of HF essentially free of HFC-1234ze via stream 220.

In another aspect of this invention, an entrainer may be added to enable separation of the HF from the HFC-1234ze, or of the HF from the HFC-1234ze and HFC-245fa and/or HFC-245eb,

For example, the mixture of HF, HFC-1234ze, HFC-245fa and/or HFC-245eb may be formed by any practical means, such was by feeding at least one of HFC-245eb or HFC-245fa over a chrome oxide catalyst at elevated temperature. The mixture of HF, HFC-1234ze, HFC-245fa and/or HFC-245eb may be fed to a distillation column. A suitable entrainer is then also fed to the distillation column, either as a separate stream or by being mixed in with the HF/HFC-1234ze/HFC-245eb and/or HFC-245fa mixture prior to feeding it to the distillation column. The distillation column is then operated under conditions sufficient to form a low-boiling azeotrope composition between the entrainer and HF, with the HF and entraitier removed as the column distillate, and the HFC-1234ze, HFC-245fa and/or HFC-245eb recovered from the column bottoms essentially free of HF. The HFC-1234ze may then be separated from the HFC-245fa and/or HFC-245eb by any usual means including conventional distillation, with the HFC-1234ze being recovered as product and with the HFC-245fa and/or HFC-245eb optionally being recycled back to the reaction step to produce HFC-1234ze.

Thus in another embodiment is provided a process for separating HF from a mixture comprising HFC-1234ze, HF, and at least one of HFC-245fa or HFC-245eb. The process comprises:
a. adding an entrainer to the mixture comprising HFC-1234ze, HF, and at least one of HFC-245fa or HFC-245eb thus forming a second mixture;
b. distilling said second mixture in a first distillation step to form a first distillate composition comprising HF and entrainer and a first bottoms composition comprising HFC-1234ze and at least one of HFC-245fa or HFC-245eb;
c. condensing said first distillate composition to form two liquid phases, being (i) an entrainer-rich phase and (ii) an HF-rich phase; and
d. recycling the entrainer-rich phase back to the first distillation step.
In another embodiment, the process may further comprise feeding the HF-rich phase to a second distillation step and forming a second distillate composition comprising an azeotrope of entrainer and HF and a second bottoms composition comprising HF essentially free of entrainer. In another embodiment, the process may further comprise recycling said second distillate composition back to the two liquid phases.

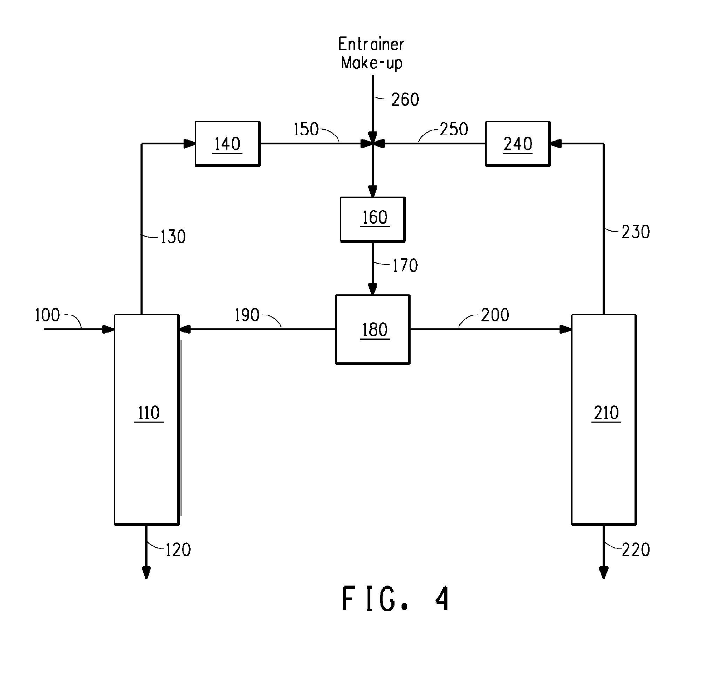

Referring now to FIG 4, a stream comprising HF, HFC-1234ze, and at least one of HFC-245fa or HFC-245eb is fed to a first distillation column 110 via stream 100. An entrainer-rich stream is also fed to this column via stream 190, Column 110 is operated under conditions to cause HF to distill overhead with the entrainer due to the influence of the low-boiling HF/entrainer azeotrope. Sufficient entrainer is fed to this first column via stream 190 much that HFC-1234ze and HFC-245fa or HFC-245eb may be obtained essentially free of entrainer and HF as the bottoms from column 110 via stream 120. The HFC-1234ze and HFC-245fa or HFC-245eb in stream 120 may then optionally be separated from each other by conventional distillation and the HFC-245fa or HFC-245eb optionally recycled back to a dehydrofluorination reactor to form HFC-1234ze. The distillate from column 110, removed via stream 130, contains essentially all of the entrainer and HF in column feeds 100 and 190 and, optionally, some HFC-245fa or HFC-245eb and/or HFC-1234ze. This first distillate stream 130 is condensed by condenser 140 to form stream 150, which is then mixed with condensed distillate stream 250 from the second distillation column and, as needed, additional fresh entrainer added via stream 260. This combined stream is sub-cooled by cooler 160 and sent via stream 170 to decanter 180 where it separates into separate entrainer-rich and HF-rich liquid fractions which are removed via streams 190 and 200, respectively, The majority of the HFC-245fa or HFC-245eb and HFC-1234ze present in the decanter partition into the entrainer-rich phase fraction. The entrainer-rich fraction is fed to the first distillation column 110 via stream 190. The HF-rich fraction from the decanter is fed via stream 200 to a second distillation column 210 containing 8 theoretical stages and operated under conditions such that a bottoms stream of HF essentially free of HFC-245fa or HFC-245eb, HFC-1234ze, and entrainer is produced and removed via stream 220, The distillate from column 210, removed via stream 230 and containing essentially all of the HFC-245fa or HFC-245eb, HFC-1234ze, and entrainer present in the column feed (stream 200) plus the HF not recovered in product stream 220, is condensed by condenser 240 and removed via stream 250. Condensed distillate stream 250 is combined with both the condensed distillate stream 150 from the first column and, as needed, fresh entrainer, added via stream 260, then cooled and fed to the decanter for further separation.

In another embodiment, a hydrofluorocarbon (HFC), which forms a homogeneous azeotrope with HF, can be separated from a mixture comprising HF, the HFC and an HFC-1234ze by azeotropic distillation using the HFC-1234ze as an entrainer, followed by separation of the HFC-1234ze and HF by azeotropic distillation using an added compound as the entrainer. HF and the HFC-1234ze are not required to be partially miscible at reduced temperatures for such a separation process to work as long as the HF-HFC-1234ze azeotrope has a lower boiling point than the HF-HFC azeotrope. For illustration purposes, the HFC-1234ze is HFC-1234ze and the HFC is HFC-245fa and/or HFC-245eb.

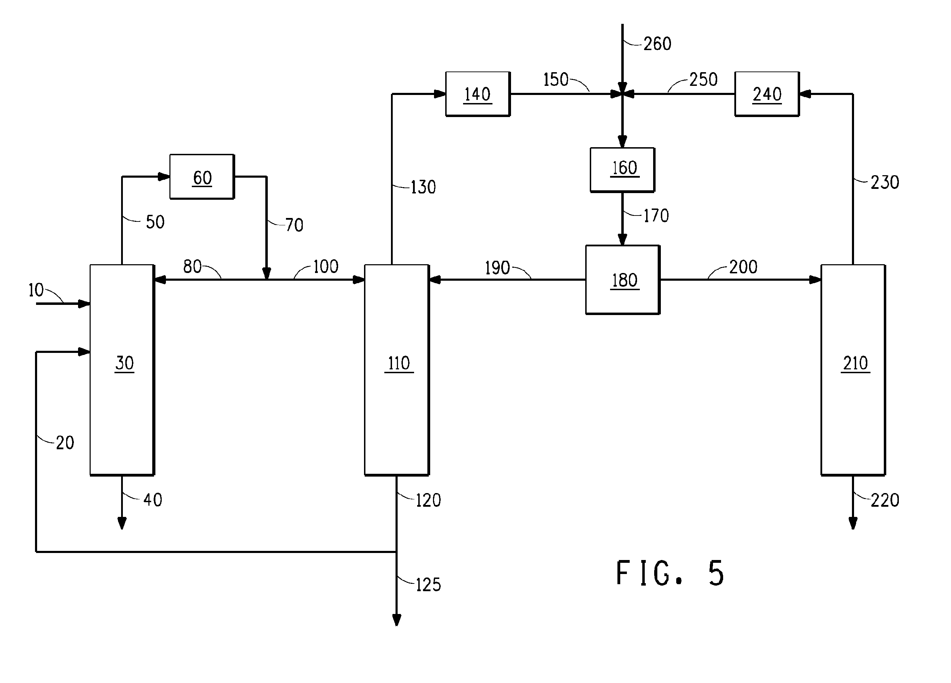

Referring now to FIG 5, a stream comprising HF, HFC-1234ze, and at least one of HFC-245fa or HFC-245eb is fed to a first distillation column 30 via stream 10, with the column operated under conditions to approach the low-boiling HF/HFC-1234ze azeotrope, which is removed as distillate via streams 50, 70, and 100. This first column can be designed and operated in such a way that the near azeotropic distillate is essentially free of HFC-245fa and/or HFC-245eb, By recycling enough supplemental HFC-1234ze from the second column bottoms to the first column via stream 20, essentially all of the HF can be distilled overhead as the HF/HFC-1234ze azeotrope such that HFC-245eb and/or HFC-245fa are obtained essentially free of HFC-1234ze and HF as the bottoms product from column 30 via stream 40. The HFC-245fa and/or HFC-245eb may then optionally be recycled back to a reactor for production of HFC-1234ze, or may be further purified and then recycled. This demonstrates the use of the HFC-1234ze as an entrainer to remove HF from an HFC.

As described for FIG 3, the distillate from the first column may be fed to a second distillation column, mixed with the distillate streams from a second and third column, cooled, and then sent to a decanter, or split between these two destinations. In this embodiment, the distillate from the first column 30 is fed via stream 100 to a second column 110. An entrainer-rich stream is also fed to this second column via stream 190. Distillation column 110 is operated under conditions such that the distillate, removed via stream 130, contains essentially all of the entrainer and HF in the column feeds 100 and 190 and produces an HFC-1234ze bottoms product essentially free of HF and entrainer which is removed via stream 120. Part of the HFC-1234ze bottoms stream 120 is recycled to the first column via stream 20, as previously described, and the rest becomes the purified HFC-1234ze product removed via stream 125. Distillate stream 130 is condensed by condenser 140 to form stream 150, which is then mixed with the condensed distillate stream 250 from the second distillation column and, as needed, fresh entrainer added via stream 260. This combined stream is cooled by cooler 160 and sent via stream 170 to decanter 180 where it separates into separate entrainer-rich and HF-rich liquid fractions, which are removed via streams 190 and 200, respectively. The majority of the HFC-1234ze present in the decanter partitions into the entrainer-rich phase fraction. The decanter entrainer-rich fraction is fed to column 110 via stream 190. The decanter HF-rich fraction is fed, via stream 200, to a third distillation column 210 operated under conditions which produce a bottoms product consisting of HF essentially free of HFC-1225zc and the entrainer, which is removed via stream 220. The distillate from column 210, which is removed via stream 230 and contains essentially all of the HFC-1234ze and entrainer present in the column feed (stream 200) and any HF not recovered in product stream 220, is condensed by condenser 240, forming stream 250. Condensed distillate stream 250 is combined with both the condensed distillate stream 150 from the second column and as needed, fresh entrainer, added via stream 260, then cooled and fed to the decanter via stream 170 for further separation.

In one embodiment, entrainers for HF separation from HFC-1234ze and optionally HFC-245fa and/or HFC-245eb include: CFC-115 (chloropentafluoroethane), CFC-114 (1,2-dichloro-1,1,2,2-tetrafluoroethane), CFC-114a (1,1-dichloro-1,2,2,2-tetrafluoroethane), HCFC-21 (dichlorofluoromethane), HCFC-124 (1-chloro-1,2,2,2-tetrafluoroethane), HCFC-124a (1-chloro-1,1,2,2-tetrafluoroethane), HCFC-133a (1-chloro-2,2,2-trifluoroethane), HCFC-142b (1-chloro-1,1-difluoroethane), HCFC-1122 (1-chloro-2,2-difluoroethylene), HFC-1123 (trifluoroethylene), 1,1-difluoroethylene (HFC-1132a), 1,1,3,3,3-pentafluoropropene (HFC-1225zc), 1,2,3,3,3-pentafluoropropene (HFC-1225ye),3,3,3-trifluoropropene (HFC-1243zf), 2,3,3,3-tetrafluoropropene (HFC-1234yf), PFC-218 (octafluoropropane), PFC-C216 (hexafluorocydopropane), cis- and trans-PFC-1318 (octafluoro-2-butene), PFC-1216 (hexafluoropropene, HFP), PFC-C318 (octafluorocyclobutane), PFC-31-10my (decafluorobutane), PFC-2316 (hexafluorobutadiene), PEVE (perfluoroethylvinylether), PMVE (perfluoromethylvinyl ether), SF₆ (sulfur hexafluoride), Cl₂ (Chlorine), cyclopropane, C₂H₆ (Ethane), propane, n-butane, isobutane, 2,2-dimethylpropane, 1-butene, isobutene, 1,3-butadiene, cis- and trans-2-butene,1-butyne, vinylacetylene, hexafluoroacetone, 1,1-difluorodimethyl ether, pentafluoroethylmethyl ether, tetrafluorodimethyl ether, and mixtures thereof.

In another embodiment, the entrainers that are effective for separation of HF from HFC-1234ze and optionally HFC-245fa and/or HFC-245eb include n-propane and ethane.

In another embodiment a process is provided for the separation of HFC-1234ze from a mixture of HFC-1234ze, HF, and at least one of HFC-245fa or HFC-245eb, said process comprising:
a) subjecting said mixture to a first distillation step, wherein additional HFC-1234ze is fed from a second distillation step, to form a first distillate comprising an azeotrope of HFC-1234ze and HF and a first bottoms composition comprising at least one of HFC-245fa or HFC-245eb;
b) feeding said first distillate to a second distillation step to form a second distillate comprising an azeotrope of HFC-1234ze and HF and a second bottoms composition comprising HFC-1234ze essentially free of HF;
c) condensing said second distillate to form two liquid phases, being i) an HF-rich phase and ii) an HFC-1234ze-rich phase; and
d) recycling the HFG-1234ze-rich phase from (c) back to the first distillation step. In another embodiment, the process may further comprise feeding the HF-rich phase to a third distillation step to form a third distillate comprising an azeotrope of HFC-1 234ze and HF and a third bottoms composition comprising HF essentially free of HFC-1234ze,

In another embodiment is provided a process for separating HF from a mixture comprising HFC-1234ze, HF, and at least one of HFC-245fa or HFC-246eb. The process comprises:
a. adding an entrainer to the mixture comprising HFC-1234ze, HF, and at least one of HFC-245fa or HFC-245eb thus forming a second mixture;
b. distilling said second mixture in a first distillation step to form a first distillate composition comprising HF and entrainer and a first bottoms composition comprising HFC-1234ze and at least one of HFC-245fa or HFC-245eb;
c. condensing said first distillate composition to form two liquid phases, being (i) an entrainer-rich phase and (ii) an HF-rich phase; and
d. recycling the entrainer-rich phase back to the first distillation step, In another embodiment, the process may further comprising feeding the HF-rich phase to a second distillation step and forming a second distillate composition comprising an azeotrope of entrainer and HF and a second bottoms composition comprising HF essentially free of entrainers. In another embodiment, the process may further comprise recycling said second distillate composition back to the two liquid phases.

In any of the embodiments as described and illustrated for FIGs 3, 5, 6, and 7, an entrainer may be added to the first distillation step to assist in removing the hydrogen fluoride from the at least one of HFC-245eb or HFC-245cb, This variation is intended to fall within the scope of the claims,

### 5. Separation of products from the co-production of HFC-1234ze and HFC-1234yf

It has been found that an HFC-1234yf/ HF azeotrope may also be used to benefit in the co-production of HFC-12-34yf and HFC-1234ze, HFC-1234yf may be produced from the dehydrofluorination of HFC-245eb (CF₃CHFCH₂F, 1,1,1,2,3-pentafluoropropane) and/or HFC-245cb (CF₃CF₂CH₃, 1,1,1,2,2-pentafluoropropane). If HFC-245eb and/or HFC-245cb and HFC-245fa and/or HFC-245eb are co-fed to a reactor containing a suitable dehydrofluorination catalyst and operating at a suitable temperature, a mixture comprising HFC-1234yf, HFC-1234ze, HF, unreacted HFC-245eb and/or HFC-245cb, and unreacted HFC-245fa, would be produced. The dehydrofluorination reaction is described in detail in PCT publication number WO2008/002500 (application number PCT/US07114645). The separation of HFC-1234ze from HFC-1234yf will be difficult by conventional distillation processes.

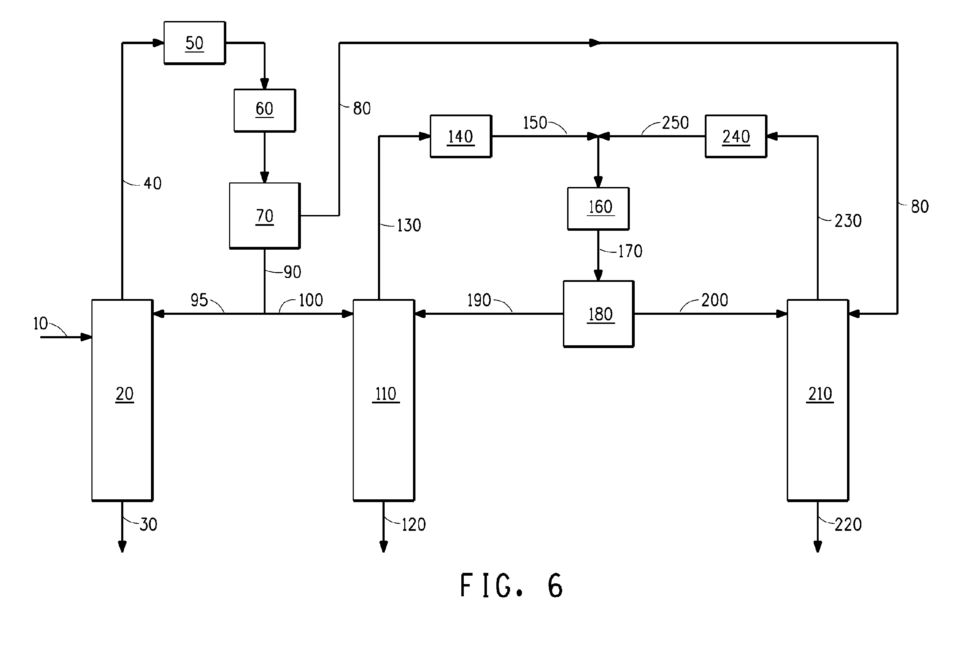

Referring now to FIG 6, the feed stream 10 leaving the dehydrofluorinatíon reactor, comprising unreacted HFC-245eb, HF, HFC-1234yf, and *E*-HFC-1234ze, is fed to a first distillation column 20. The column 20 is operated such that essentially all of the HFC-245eb in the feed, most of the *E*-HFC-1234ze, and a relatively small amount of the HFC-1234yf in the feed are removed, essentially free of HF, from the bottom of the column via stream 30. Stream 30 may optionally undergo further purification or be recycled to the dehydrofluorination reactor. Essentially all of the HF, the majority of the HFC-1234yf and, optionally, varying amounts of HFC-245eb and/or *E*-HFC-1234ze are removed from the top of the column via stream 40, condensed in condenser 50, cooled in cooler 60, and fed to a first decanter 70 where a first HF-rich phase and a first fluoroolefin-rich phase fractions are formed. The first fluoroolefin-rich phase fraction is removed via stream 90 and divided into two portions. The first portion is returned to the first column 20 as reflux via stream 95 and the remaining portion is sent to a second distillation column 110 via stream 100. The flow rate of stream 95 is adjusted such that stream 95 contains enough additional HFC-1234yf to enable essentially all of the HF present in stream 10 to be distilled from the top of column 20 due to the existence of the low-boiling HF/HFC-1234yf azeotrope.

Stream 100 (a portion of the first fluoroolefin-rich phase from decanter 70) is fed to the second distillation column 110 where it is separated into a fluoroolefin bottoms product that is essentially free of HF, removed via stream 120, and a distillate composition near to the HF/HFC-1234yf azeotrape, removed via stream 130.

The first HF-rich phase fraction (from the first decanter 70) is fed to a third distillation column 210 via stream 80 along with the second HF-rich phase fraction from a second decanter 180 via stream 200. Both feeds (streams 80 and 200) to the third distillation column 210 have compositions containing excess HF relative to the HF/HFC-1234yf azeotrope so that an HF bottoms product essentially free of HFC-1234yf may be obtained in column 210 and removed via stream 220. The distillate from the third column has a composition near to the HF/HFC-1234yf azeotrope and is removed via stream 230. The distillates (streams 130 and 230) from columns 110 and 210 are condensed in condensers 140 and 240, forming streams 150 and 250, respectively, mixed together, and sent first to a second cooler 160 and then to the second decanter 180 where the second fluoroolefin-rich and second HF-rich liquid phase fractions are formed. The second fluoroolefin-rich fraction is removed from decanter 180 via stream 190 and fed to the second column 110 for further separation. The second HF-rich fraction is removed from decanter 180 via stream 200 and fed to the third column 210 for further separation.

In another embodiment, column 20 may be operated under conditions that cause essentially all of the *E*-HFC-1234ze in feed stream 10 to distill with the HF and HFC-1234yf, thereby allowing an HFC-245eb stream, essentially free of HF, HFC-1234yf, and *E*-HFC-1234ze to be removed from the bottom of the column via stream 30.

In another embodiment, column 20 may be operated under conditions to cause essentially all of the *E*-HFC-1234ze in feed stream 10 to be taken out of column 20 at the bottom. The result for this embodiment, would be the production of HFC-1234yf essentially free of *E-*HFC-1234ze.

### EXAMPLES

The concepts described herein will be further described in the following examples.

### EXAMPLE 1

### Dehydrofluorinatlon of HFC-245fa to HFC-1234ze (E and Z isomers) over carbonaceous catalyst

To a Hastelloy nickel alloy reactor (1,0" OD X 0.854" ID X 9.5" L) was charged 14.32 g (25 mL) of spherical (8 mesh) three dimensional matrix porous carbonaceous material prepared substantially as described in U.S. Patent No, 4,978,649. The packed portion of the reactor was heated by a 5" X 1" ceramic band heater clamped to the outside of the reactor. A thermocouple, positioned between the reactor wall and the heater measured the reactor temperature. After charging the reactor with the carbonaceous material nitrogen (10 mL/min) was passed through the reactor and the temperature was raised to 200 °C during a period of one hour and maintained at this temperature for an additional 4 hours. The reactor temperature was then raised to the desired operating temperature and a flow of HFC-245fa and nitrogen was started through the reactor.

A portion of the total reactor effluent was sampled on-line for organic product analysis using a gas chromatograph equipped with a mass selective detector (GC-MS). The bulk of the reactor effluent containing organic products and also inorganic acid, such as HF, was treated with aqueous caustic for neutralization.

Results obtained in GC area% are summarized in Table 1,

**TABLE 1**

| Reactor Temp. (°C) | HFC-245fa feed (mL/min) | N₂ feed (mL/min) | Mole Percent | | | |
|---|---|---|---|---|---|---|
| | | | *E*-HFC-1234ze | *Z*-HFC-1234ze | HFC-245fa | Unknowns |
| 200 | 10 | 20 | 0.1 | ND | 99.6 | 0.3 |
| 250 | 10 | 20 | 0.8 | ND | 99.0 | 0.2 |
| 300 | 10 | 20 | 8,9 | ND | 90.9 | 0.2 |
| 350 | 10 | 10 | 31.6 | 5.7 | 62.3 | 0.4 |
| 350 | 10 | 5 | 42.4 | 8.7 | 48.3 | 0.6 |

| | | | | | | |
|---|---|---|---|---|---|---|
| ND = not detected | | | | | | |

### EXAMPLE 2

### Dehydrofluorination of HFC-245fa to HFC-1234ze (E and Z isomers) over fluorided alumina catalyst

A 15 in x 3/8 in Hastelloy tube was filled with 7.96 grams (13cc) of gamma-alumina ground to 12-20 mesh. The catalyst was activated by heating at 200°C for 15 minutes under a nitrogen purge (50 sccm, 8.3x10⁻⁷ m³/s). The temperature was raised to 325°C for 10 minutes, to 400°C for 20 minutes, and then lowered to 300°C for 60 minutes. The nitrogen was lowered to 35 sccm (5.8x10⁻⁷ m³/s) and anhydrous HF vapor was fed at 12 sccm (2.0x10⁻⁷ m³/s) for 35 minutes. The temperature was then raised to 325°C for 60 minutes, to 350°C for 60 minutes, to 375°C for 90 minutes, to 400°C for 30 minutes, and to 425°C for 40 minutes. The nitrogen was then lowered to 25 sccm (4.2x10⁻⁷ m³/s) and the HF raised to 20 sccm (3.3 x10⁻⁷ m²/s) for 20 minutes. The nitrogen was then lowered to 15 sccm (2.5x10⁻⁷ m³/s) and the HF raised to 28 sccm (4.7 x10⁻⁷ m³/s) for 20 minutes. The nitrogen was then lowered to 5 sccm (8.3x10⁻⁸ m³/s) and the HF raised to 36 sccm (6.0 x10⁻⁷ m³/s) for 20 minutes. The nitrogen was then shut off, and the HF raised to 40 sccm (6.7 x10⁻⁷ m³/s) for 121 minutes.

The temperature of the reactor was set to 375 °C, and HFC-245fa was fed at a flow rate of 5.46 mL/hour (20.80 sccm, 3.5 x10⁻⁷ m³) and a nitrogen flow rate of 5.2 sccm (8.7 x10⁻⁸ m³). The effluent was analyzed by GC and the results are shown in Table 2.

**TABLE 2**

| **Component** | **GC Area %.** |
|---|---|
| *E*-HFC-1234ze | 71.4 |
| HFC-245fa | 15.2 |
| *Z*-HFC-1234ze | 12-1 |
| unknown | 1.3 |

### EXAMPLE 3

### Azeotropic distillation for the separation of E-HFC-1234ze from HF without an entrainer

Example 3 demonstrates that HF may be separated from *E*-HFC-1234ze by azeotropic distillation with no entrainer. Referring now to FIG 1, a composition comprising HF and *E*-HFC-1234ze is fed to a first column 110 via stream 100. This first column contains 8 theoretical stages and is operated under appropriate conditions to approach the low-boiling HF/ *E-*HFC-1234ze azeotrope. Because HF is being fed to this first column in excess of that needed to form the azeotrope with *E*-HFC-1234ze, HF is recovered as a product stream from the bottom of the column via stream 120, while a composition near to the HF/ *E*-HFC-1 234ze azeotrope is recovered as distillate via stream 130. Stream 130 is condensed in condenser 140, mixed with the nearly azeotropic composition recycled from the second column via stream 250 and the combined stream is sub-cooled in cooler 160 and sent to decanter 180 where the combined stream 170 separates into a separate HF-rich stream 190 and an *E*-HFC-1234ze-rich stream 200. Stream 190 is recycled to the first column as reflux. Stream 200 is fed to the top stage of a second distillation column 210, containing 19 theoretical stages and operated under conditions to approach the HF/ *E*HFC-1234ze azeotrope. Because *E*-HFC-1234ze is being fed to this second column in excess of that needed to form the low-boiling HF/ *E*-HFC-1234ze azeotrope, *E*-HFC-1234ze is recovered as a product stream from the bottom of the column via stream 220 while a composition close to the HF/ *E*-HFC-1234ze azeotrope is recovered as distillate via stream 230. Stream 230 is condensed in condenser 240, mixed with the nearly azeotropic composition from the first column via stream 150 and fed to cooler 160 and then decanter 180.

The data in Table 3 were calculated using measured and calculated thermodynamic properties,

**TABLE 3**

| Component or variable | First dist. col. feed | First column distillate | First dist. col. bottom (HF product) | HF rich phase (from decanter) | *E*-HFC-1234ze rich phase (from decanter) | Second distillate | Second dist. col. Bottom (*E*-HFC-1234ze product) |
|---|---|---|---|---|---|---|---|
| Stream No. | **100** | **130** | **120** | **190** | **200** | **230** | **220** |
| HF. wt% | 14.9 | 8.0 | 100 | 15.5 | 1.7 | 5.5 | 1ppm |
| *E*-HFC-1234ze, wt% | 85.1 | 92.0 | 10ppm | 84.5 | 98.3 | 94.5 | 100 |
| Temp. °C | 30.0 | 48.9 | 102 | -40.0 | -40.0 | 49.2 | 54.0 |
| Pres, psia | 165 | 160 | 160 | 159 | 159 | 160 | 160 |
| MPa | 1.14 | 1.10 | 1.10 | 1.09 | 1.09 | 1.10 | 1.10 |

### EXAMPLE 4

### Azeotropic distillation for the separation of E-HFC-1234ze from HF using propane as the entrainer

Example 4 demonstrates that HF may be separated from *E*-HFC-1234ze by azeotropic distillation using propane as the entrainer.

Referring now to FIG 2, a composition comprising HF and *E*-HFC-1234ze is fed to a first column 110 containing 9 theoretical stages via stream 100. An HF-rich and propane-lean composition is also fed to the top stage of column 110 via stream 190. Because the combined amount of HF in streams 100 and 190 is in excess of that needed to form the low-boiling HF/propane and HF/ *E*-HFC-1234ze azeotropes, HF is recovered as a product stream essentially free of both *E*-HFC-1234ze and propane from the bottom of column 110 via stream 120. A ternary composition enriched in *E*-HFC-1234ze and propane relative to the combined feeds 100 and 190 is recovered as the distillate via stream 130. Stream 130 is condensed by condenser 140 forming stream 150 and mixed with both the condensed distillate stream 250 from a second distillation column and, as needed, additional propane added via stream 260. Combined streams 150, 250, and 260 are sent to cooler 160 and then to decanter 180 where the sub-cooled liquid stream 170 separates into HF-rich and propane-rich liquid phase fractions which are removed via streams 190 and 200, respectively. The *E*-HFC-1234ze present in the decanter primarily distributes into the propane-rich liquid phase fraction. Stream 190 is recycled to the first column. The HF-lean liquid phase fraction in the decanter is fed to the top stage of a second distillation column 210 via stream 200. Column 210 contains 25 theoretical stages. Because the amount of *E*-HFC-1234ze in stream 200 is in excess of that needed to form the low-boiling propane/ *E*-HFC-1234ze, *E*-HFC-1234ze/HF, and propane/ *E*-HFC-1234ze/HF azeotropes, i.e., the composition of stream 200 lies in the distillation region bounded by these three azeotrope compositions and pure *E*-HFC-1234ze, *E*-HFC-1234ze is recovered as a product stream essentially free of both HF and propane from the bottom of column 210 via stream 220. A ternary composition enriched in propane and HF relative to stream 200 and in the same distillation region leaves the top of the second column as the distillate via stream 230. Stream 230 is condensed by condenser 240, forming stream 250, and combined with streams 150 and 260 as previously described.

The data in Table 4 were calculated using measured and calculated thermodynamic properties

**TABLE 4**

| Component or variable | First dist. col. feed | First distillate | First dist. col. bottom (HF product) | HF rich phase (from decanter) | Propane rich phase (from decanter) | Second distillate | Second dist. col. Bottom (*E*-HFC-1234ze product) |
|---|---|---|---|---|---|---|---|
| Stream No. | **100** | **130** | **120** | **190** | **200** | **230** | **220** |
| HF, wt% | 14.9 | 8.0 | 100 | 15.6 | 0.79 | 1.3 | <1ppm |
| HFC-1234ze, wt% | 85.1 | 90.4 | 1 ppm | 81.2 | 76.2 | 59.3 | 100 |
| Propane, w% | 0 | 1.6 | <1ppm | 3.2 | 23.0 | 39.3 | 1 ppm |
| Temp, °C | 25.0 | 35.4 | 88.6 | -20.0 | -20.0 | 18.4 | 41.2 |
| Pres, psia | 115 | 115 | 115 | 115 | 115 | 115 | 115 |
| MPa | 0.79 | 0.79 | 0.79 | 0.79 | 0.79 | 0.79 | 0.79 |

### EXAMPLE 5

This Example shows one way in which HF may be separated from *E*-HFC-1234ze and HFC-245fa. The composition of the feed mixture in this example is such as one might obtain from a dehydrofluorination reactor operated with partial conversion, i.e., it contains equimolar amounts of HF and *E*-HFC-1234ze.

Referring now to FIG 4, a stream comprising HF, *E*-HFC-1234ze, and HFC-245fa is fed to a first distillation column 110 via stream 100. An entrainer-rich stream is also fed to this column via stream 190. In this example, propane is used as the entrainer.

Column 110 contains 34 theoretical stages and is operated under conditions to cause HF to distill overhead with the entrainer due to the influence of the low-boiling HF/propane azeotrope. Sufficient propane is fed to this first column via stream 190 such that *E*-HFC-1234ze and HFC-245fa may be obtained essentially free of propane and HF as the bottoms from column 110 via stream 120. The *E*-HFC-1234ze and HFC-245fa in stream 120 may then optionally be separated from each other by conventional distillation and the HFC-245fa optionally recycled back to a dehydrofluorinatlon reactor to form HFC-1234-ze. The distillate from column 110, removed via stream 130, contains essentially all of the propane and HF in column feeds 100 and 190 and, optionally, some HFC-245fa and/or *E*-HFC-1234ze. This first distillate stream 130 is condensed by condenser 140 to form stream 150, which is then mixed with condensed distillate stream 250 from the second distillation column and, as needed, additional fresh propane added via stream 260. This combined stream is sub-cooled by cooled 160 and sent via stream 170 to decanter 180 where it separates into separate entrainer-rich and HF-rich liquid fractions which are removed via streams 190 and 200, respectively. The majority of the HFC-245fa and *E*-HFC-1234ze present in the decanter partition into the propane-rich phase fraction. The propane-rich fraction is fed to the first distillation column 110 via stream 190. The HF-rich fraction from the decanter is fed via stream 200 to a second distillation column 210 containing 8 theoretical stages and operated under conditions such that a bottoms stream of HF essentially free of HFC-245fa, *E*-HFC-1234ze, and propane is produced and removed via stream 220. The distillate from column 210, removed via stream 230 and containing essentially all of the HFC-245fa, *E*-HFC-1234ze, and propane present in the column feed (stream 200) plus the HF not recovered in product stream 220, is condensed by condenser 240 and removed via stream 250. Condensed distillate stream 250 is combined with both the condensed distillate stream 150 from the first column and, as needed, fresh entrainer, added via stream 260, then cooled and fed to the decanter for further separation.

The data in Table 5 were obtained by calculation using measured and calculated thermodynamic properties.

**TABLE 5**

| Component or variable | Feed | First col btm | First Dist | Propane-rich phase | HF-rich phase | Second col btm | Second Dist |
|---|---|---|---|---|---|---|---|
| Stream # | 100 | 120 | 130 | 190 | 200 | 220 | 230 |
| HF, wt% | 6.4 | <1 ppm | 3.2 | 0.2 | 43.7 | 100 | 34.1 |
| HFC-245fa, wt% | 57.1 | 61.0 | 11.5 | 11.9 | 11.1 | 2 ppm | 13.0 |
| *E*-HFC-1234ze, wt% | 36.5 | 39.0 | 29.1 | 30.0 | 43.6 | <1 ppm | 51.0 |
| Propane wt% | 0 | 1 ppm | 56.2 | 57.9 | 1.6 | <1 ppm | 1.9 |
| Temp, °C | 30.0 | 59.5 | 17.2 | -35.0 | -35.0 | 88.9 | 66.5 |
| Pres, psia | 165 | 116 | 115 | 115 | 115 | 116 | 115 |
| MPa | 1.14 | 0.80 | 0.79 | 0.79 | 0.79 | 0.80 | 0.79 |

### EXAMPLE 6

This Example shows how HF, *E*-HFC-1234ze, and HFC-245eb may be separated using *E*-HFC-1234ze as an entrainer. One possible source for such a mixture is in an HFC-245eb dehydrofluorination process operated with partial conversion.

Referring now to FIG 3, a stream comprising HF, *E*-HFC-1234ze, and HFC-245eb is fed to the 33^{rd} stage of a first distillation column 30 containing 40 theoretical stages via stream 10, with the column operated under conditions to approach the low-boiling HF/ *E*-HFC-1234ze azeotrope at the top of the column, which is removed as vapor from the top of the column via stream 50. Enough supplemental *E*-HFC-1234ze is recycled from the second column bottoms to the 12^{th} stage of the first column 30 via stream 20 to enable essentially all of the HF to be removed from the HFC-245eb. The HFC-245eb obtained as the bottoms product from the first column 30 via stream 40 is essentially free of *E*-HFC-1234ze and HF and as such may be recycled to the dehydrofluorination reaction process

The near HF/ *E*-HFC-1234ze azeotropic composition in vapor stream 50 is condensed and divided into reflux 80 and distillate 90 streams. In FIG 3, distillate stream 90 may be fed to a second distillation column 110 via stream 100 or may be mixed with distillate streams 150 and 250 and sent to cooler 160, or be divided between these two destinations.

For this example, none of distillate stream 90 is fed directly to column 110 via stream 100. Instead stream 90 via stream 260 is mixed with distillate streams 150 and 250 from the second and third columns, respectively, and sent to cooler 160, forming sub-cooled stream 170, which is fed to decanter 180. In the decanter, stream 170 separates into *E*-HFC-1234ze-rich and HF-rich liquid fractions, which are removed as streams 190 and 200, respectively. The *E*-HFC-1234ze-rich stream from the decanter is fed via stream 190 to a second distillation column 110 containing 19 theoretical stages and operated under conditions to approach the *E*-HFC-1234ze / HF azeotrope, which is distilled overhead as distillate stream 130, condensed in condenser 140, and mixed with the distillates from the first and third columns via stream 150. Column 110 produces a bottoms stream of *E*-HFC-1234ze essentially free of HF via stream 120. Part of the *E*-HFC-1234ze bottoms stream 120 is recycled to the first column via stream 20, as previously described, and the rest becomes the purified *E*-HFC-1234ze product removed via stream 125. The HF-rich stream from the decanter is fed via stream 200 to a third distillation column 210 containing 9 theoretical stages and operated under conditions to approach the *E*-HFC-1234ze /HF azeotrope, which is distilled overhead as distillate as stream 230 which is condensed in condenser 240 and mixed with the distillates from the first and second columns via stream 250. Column 210 produces a bottoms stream of HF essentially free of *E-*HFC-1234ze and HFC-245ebvia stream 220.

The data in Table 6 were obtained by calculation using measured and calculated thermodynamic properties.

**TABLE 6**

| Component or variable | Feed | First btms | First Dist | Second btms | Second Dist | *E*-HFC-1234ze -rich phase | HF-rich phase | Third btms | Third Dist |
|---|---|---|---|---|---|---|---|---|---|
| Stream # | 10 | 40 | 90 | 120 | 130 | 190 | 200 | 220 | 230 |
| HF, wt% | 4.0 | <1 ppm | 4.7 | 1 ppm | 5.9 | 1.7 | 15.5 | 100 | 7.8 |
| HFC-245eb, wt% | 71.3 | 100 | 1 ppm | 1 ppm | <1 ppm | <1 ppm | <1 ppm | <1 ppm | <1 ppm |
| *E*-HFC-1234ze, wt% | 24.7 | 3 ppm | 95.3 | 100 | 94.1 | 98.3 | 84.5 | 1 ppm | 92.2 |
| Temp, °C | 27.7 | 61.1 | 12.6 | 37.9 | 33.7 | -40.0 | -40.0 | 84.9 | 33.5 |
| Pres, psia | 61 | 55 | 55 | 105 | 105 | 105 | 105 | 105 | 105 |
| MPa | 0.42 | 0.38 | 0.38 | 0.73 | 0.73 | 0.73 | 0.73 | 0.73 | 0.73 |

### EXAMPLE 7

This example shows how HFC-245fa that forms an azeotrope with HF and *E*-HFC-1234ze that is partially miscible with and forms an azeotrope with HF can both be separated from a mixture comprising HF, HFC-245fa and *E*-HFC-1234ze by azeotropic distillation using n-propane as an added entrainer.

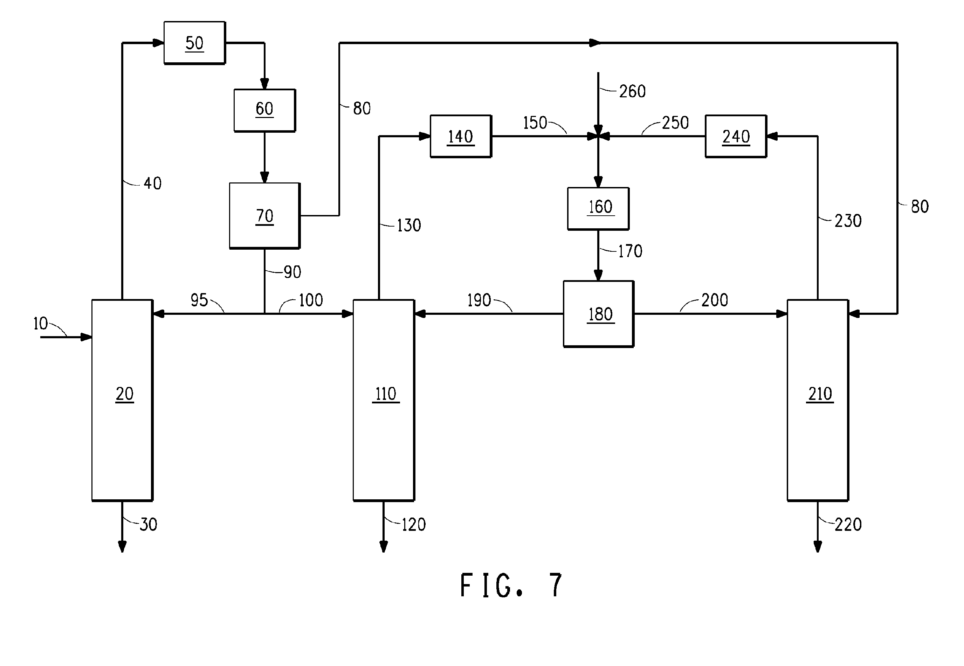

Referring now to FIG 7, a mixture comprising HF, *E*-HFC-1234ze, and HFC-245fa is fed to the 25th stage from the top of a first distillation column 20 containing 40 theoretical stages via stream 10. The HFC-245fa present in stream 10 is separated from HF and *E*-HFC-1 234ze by azeotropic distillation in this first distillation column 20 using the *E*-HFC-1234ze in the feed mixture as the entrainer However, the equimolar HF/ *E*-HFC-1234ze mixture from a dehydrofluorination process does not contain enough *E*-HFC-1234ze to cause all of the HF to distill overhead. Consequently, supplemental *E*-HFC-1234ze in an amount sufficient to cause all of the HF to distill away from the HFC-245fa is added via a second, *E*-HFC-1234ze-enriched stream 95 that is added as reflux to the top of the first column 20. Column 20 is operated under conditions to approach the low-boiling HF/ *E*-HFC-1234ze azeotrope, which is removed as distillate via stream 40. An essentially HF-free mixture comprising HFC-245fa is removed from the bottom of the column via stream 30 and may, if desired, be returned to a dehydrofluorination reaction step.

Vapor stream 40 is condensed and cooled in a first condenser 50 and a first cooler 60, and then sent to first decanter 70 where HF-rich and *E*-HFC-1234ze-rich liquid phase fractions are formed and removed via streams 80 and 90, respectively. Part of the *E*-HFC-1234ze-rich decanter liquid phase fraction removed via stream 90 is returned to the first column as reflux and as the source of supplemental *E*-HFC-1234ze previously described via stream 95 and the remaining portion is fed to the top stage of a second distillation column 110 (containing 19 theoretical states) via stream 100 where it is separated into an *E*-HFC-1 234ze bottoms product, removed via stream 120, that is essentially free of HF and a distillate composition removed via stream 130. The first decanter's HF-rich phase fraction is fed to a third distillation column 210 via stream 80. Both feeds (streams 80 and 200) to the third column have compositions containing excess HF relative to the azeotropes so that an HF bottoms product essentially free of *E*-HFC-1234ze and propane may be obtained in column 210 and removed via stream 220. The distillate from the third column is enriched in *E*-HFC-1234ze and propane relative to the combined feeds 80 and 200, and is removed via stream 230. As in earlier examples, the distillates (streams 130 and 230) from columns 110 and 210 are condensed in condensers 140 and 240, forming streams 150 and 250, respectively, mixed together, along with, as needed additional propane added via stream 260and sent first to a second cooler 160 and then to a second decanter 180 where separate propane-rich and HF-rich liquid phase fractions are formed. The propane-rich fraction is removed from decanter 180 via stream 190 and fed to the second column 110 for further separation. The HF-rich fraction is removed from decanter 180 via stream 200 and fed to the third column 210 for further separation.

The data in Table 7 were obtained by calculation using measured and calculated thermodynamic properties.

**TABLE 7**

| Component or variable | Feed | First btm | First HF-rich phase | *E*-HFC-1234ze-rich phase | See Btm | Sec. Dist | Propane -rich phase | Second HF-rich phase | Third btms | Third dist |
|---|---|---|---|---|---|---|---|---|---|---|
| Stream # | 10 | 30 | 80 | 90 | 120 | 130 | 190 | 200 | 220 | 230 |
| HF, wt% | 5.0 | <1ppm | 15.5 | 1.7 | <1ppm | 0.25 | 0.2 | 41.4 | 100 | 8.3 |
| HFC-245fa. wt% | 66.7 | 100 | <1ppm | <1ppm | <1ppm | <1ppm | <1ppm | <1 ppm | <1ppm | <1pp m |
| *E*-HFC-1234ze, wt% | 28.3 | <1ppm | 84.5 | 98.3 | 100 | 41.3 | 48.4 | 57.0 | 1ppm | 91.4 |
| Propane, wt% | 0 | 0 | 0 | 0 | 10 ppm | 58.4 | 51.4 | 1.6 | <1ppm | 0.32 |
| Temp, °C | 37.0 | 53.6 | -40.0 | -40.0 | 16.2 | -8.4 | -40.0 | -40.0 | 60.7 | 12.7 |
| Pres, psia | 55 | 55 | 55 | 55 | 55 | 55 | 55 | 55 | 55 | 55 |
| MPa | 0.38 | 0.38 | 0.38 | 0.38 | 0.38 | 0.38 | 0.38 | 0.38 | 0.38 | 0.38 |

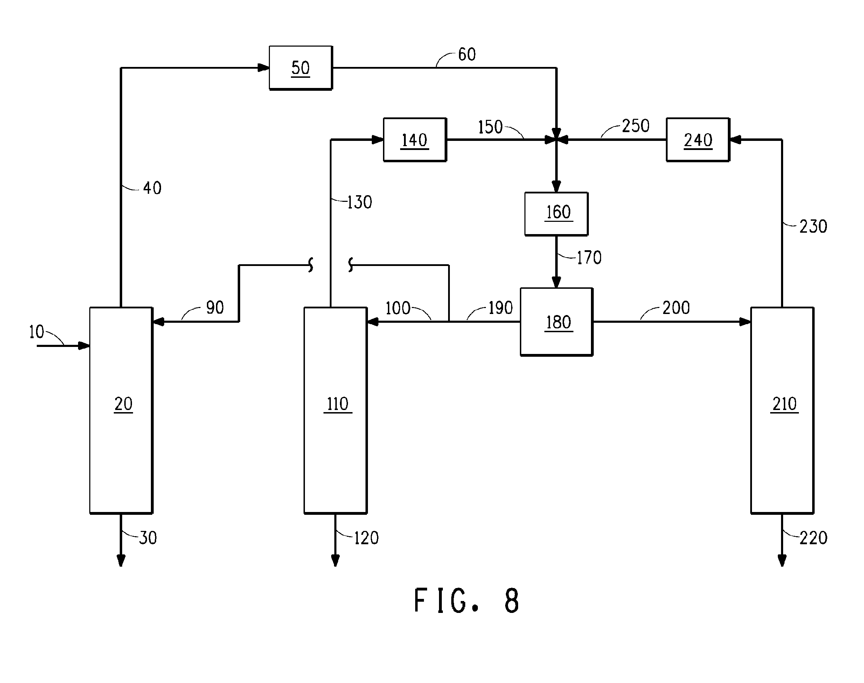

In other embodiments of the invention, (a) condensers 140 and 240 may be combined into a single unit, (b) coolers 60 and 160 can be combined into a single unit and decanters 70 and 180 can be combined into a unit, as shown in FIG 8, or (c) the three condensers 50, 140 & 240 can be combined into a single unit, coolers 60 and 160 can be combined into a single unit, and decanters 70 and 180 can be combined into a unit.

## Claims

1. A process for separating a mixture comprising HF and *E*-HFC-1234ze, said process comprising
a. feeding the composition comprising HF and *E*-HFC-1234ze to a first distillation column;
b. removing an azeotrope composition comprising HF and *E-*HFC-1234ze as a first distillate and either i) HF or ii) *E*-HFC-1234ze as a first column bottoms composition:
c. condensing the first distillate to form 2 liquid phases, being i) an HF-rich phase and ii) an *E*-HFC-1234ze-rich phase; and
d. recycling a first liquid phase enriched in the same compound that is removed as the first column bottoms, said first liquid phase being either i) HF-rich phase or ii) E-HFC-1234ze-rich phase, back to the first distillation column.

2. The process of claim 1, further comprising feeding a second liquid phase not recycled in step (d), said second liquid phase being either i) HF-rich phase or ii) *E*-HFC-1234ze-rich phase, to a second distillation column, and recovering the compound not recovered in step (b) as the first column bottoms composition as the second column bottoms composition.

3. A process as claimed in claim 1 for separating *E*-HFC-1234ze from a mixture comprising hydrogen fluoride and said *E*-HFC-1234ze, wherein said *E*-HFC-1234ze is present in a concentration greater than the azeotrope concentration for hydrogen fluoride and said *E*-HFC-1234ze, said process comprising:
a. feeding said mixture comprising hydrogen fluoride and said *E*-HFC-1234ze to a first distillation column;
b. removing an azeotrope composition comprising hydrogen fluoride and *E*-HFC-1234ze as a first distillate from the first distillation column;
c. recovering *E*-HFC-1234ze essentially free of hydrogen fluoride from the bottom of the first distillation column;
d. condensing the azeotrope composition to form two liquid phases, being i) a hydrogen fluoride-rich phase and ii) an *E*-HFC-1234ze-rich phase; and
e. recycling the *E*-HFC-1234ze-rich phase to the first distillation column.

4. The process of claim 3 further comprising:
a. feeding the hydrogen fluoride-rich phase to a second distillation column, and
b. recovering hydrogen fluoride containing less than 100 ppm of *E*-HFC-1234ze from the bottom of the second distillation column.

5. A process as claimed in claim 1 for separating hydrogen fluoride from a mixture comprising hydrogen fluoride and an *E*-HFC-1234ze, wherein hydrogen fluoride is present in a concentration greater than the azeotrope concentration for hydrogen fluoride and said *E*-HFC-1234ze, said process comprising;
a. feeding said mixture comprising hydrogen fluoride and *E-*HFC-1234ze to a first distillation column,
b. removing an azeotrope or azeotrope-like composition comprising *E*-HFC-1234ze and HF as a distillate from the first distillation column;
c. recovering hydrogen fluoride containing less than 100 ppm of *E*-HFC-1234ze from the bottom of the first distillation column;
d. condensing the azeotrope composition to form two liquid phases, being an *E*-HFC-1234ze-rich phase and a hydrogen fluoride-rich phase; and
e. recycling the HF-rich phase to the first distillation column.

6. The process of claim 5, further comprising:
a. feeding the *E*-HFC-1234ze-rich phase to a second distillation column; and
b. recovering *E*-HFC-1234ze containing less than 100 ppm of hydrogen fluoride from the bottom of the second distillation column.

7. A process for the purification of an *E*-HFC-1234ze from a mixture comprising *E*-HFC-1234ze and HF, wherein said *E*-HFC-1234ze is present in said mixture in a concentration greater than the azeotrope concentration for said *E*-HFC-1234ze and HF, said process comprising:
a. adding an entrainer to the mixture comprising *E*-HFC-1234ze and HF thus forming a second mixture;
b. distilling said second mixture in a first distillation step to form a first distillate composition comprising HF, *E*-HFC-1234ze, an entrainer, and a first bottoms composition comprising *E*HFC-1234ze;
c. condensing said first distillate composition to form two liquid phases, being i) an HF-rich phase and ii) an entrainer-rich phase; and
d. optionally recycling the entrainer-rich phase back to the first distillation step.

8. A process for the purification of HF from a mixture comprising an *E-*HFC-1234ze and HF, wherein HF is present in a concentration greater than the azeotrope concentration for HF and said *E*-HFC-1234ze, said process comprising:
a. adding an entrainer to the mixture comprising *E*-HFC-1234ze and HF thus forming a second mixture;
b. distilling said second mixture in a first distillation step to form a first distillate composition comprising an HF, entrainer, and *E*-HFC-1234ze, and a first bottoms composition comprising HF;
c. condensing said first distillate composition to form two liquid phases, being i) an entrainer-rich phase and ii) an HF-rich phase; and
d. optionally recycling the HF-rich phase back to the first distillation step.

9. The process of claim 8 further comprising feeding the entrainer-rich phase of step (c) to a second distillation step and forming a second distillate composition comprising an azeotrope of entrainer and HF and a second bottoms composition comprising HF containing less than 100 ppm of entrainer.

10. A process for the preparation of *E*-HFC-1234ze from a mixture of *E*-HFC-1234ze, and at least one of HFC-245fa or HFC-245eb, sait process comprising:
a) subjecting said mixture to a first distillation step, wherein additional *E*-HFC-1234ze is fed from a second distillation step, to form a first distillate comprising an azeotrope of *E*-HFC-1234ze and HF and a first bottoms composition comprising at least one of HFR-245fa or HFC-245eb;
b) feeding said first distillate to a second distillation step to form a second distillate comprising an azeotrope of *E*-HFC-1234ze and HF and a second bottoms composition comprising *E*-HFC-1234ze containing less than 100 ppm of HF;
c) condensing said second distillate to form two liquid phases, being i) an HF-rich and ii) an *E*-HFC-1234ze-rich phase; and
d) recycling the *E*-HFC-1234ze-rich phase from (c) back to the first distillation step.

11. A process for separating HF from a mixture comprising *E*-HFC-1234ze, HF, and at least one of HFC-245fa or HFC-245eb, said process comprising:
a. adding an entrainer to the mixture comprising *E*-HFC-1234ze, HF, and at least one of HFC-245fa or HFC-245eb thus forming second mixture;
b. distilling said second mixture in a first distillation step to form a first distillate composition comprising HF and entrainer and a first bottoms composition comprising *E*-HFC-1234ze and at least one of HFC-245fa or HFC-245eb;
c. condensing said first distillate composition to form two liquid phases, being i) an entrainer-rich phase and ii) an HF-rich phase; and
d. recycling the entrainer-rich phase back to the first distillation step.

12. The process of claim 11 further comprising recycling said second distillate composition back to the two liquid phases.

13. The process of claim 7, 8 or 11, wherein said entrainer is selected from the group consisting of :
a. hydrocarbon entrainers comprising at least one compound selected from the group consisting of: methane, ethane, ethylene, acetylene, vinylacetylene, n-propane, propylene, propyne, cyclopropane, cyclopropene, propadiene, n-butane, isobutane, 1-butene, isobutene, 1,3-butadiene, 2,2-dimethylpropane, cis-2-butene, trans-2-butene, 1-butyne, n-pentane, isopentane, neopentane, cyclopentane, 1-pentene, 2-pentene and mixtures thereof;
b. chlorocarbon entrainers selected from the group consisting of methylene chloride, methyl chloride, and mixtures thereof;
c. chlorofluorocarbon (CFC) entrainers comprising at least one compound selected from the group consisting of: dichlorodifluoromethane (CFC-12), 2-chloro-1,1,2-trifluoroethylene, chloropentafluoroethane (CFC-115), 1,2-dichloro-1,1,2,2-tetrafluoroethane (CFC-114), 1,1-dichloro-1,2,2,2-tetrafluoroethane (CFC-114a), 1,1,2-trichloro-1,2,2-trifluoroethane (CFC-113), 1,1,1-trichloro-2,2,2-trifluoroethane (CFC-113a), 1,1,2-trichloro-1,2,3,3,3-pentafluoropropane (CFC-215bb), 2,2-dichloro-1,1,1,3,3,3-hexafluoropropane (CFC-216aa), 1,2-dichloro-1,1,2,3,3,3-hexafluoropropane (CFC-216ba), 2-chloro-1,1,1,2,3,3,3-heptafluoropropane (CFC-217ba), 2-chloro-1,1,3,3,3-pentafluoropropene (CFC-121xc), and mixtures thereof;
d. hydrochlorofluorocarbon (HCFC) entrainers comprising at least one compound selected from the group consisting of: dichlorofluoromethane (HFCF-21), 1-1-dichloro-3,3,3-trifluoroethane (HCFC-123), 1,1-dichloro-1-fluoroethane (HCFC-141b), 2-chloro-1,1,1,2-tetrafluoroethane (HCFC-124), 1-chloro-1,1,2,1-tetrafluoroethane (HCFC-124a) 2-chloro-,1,1,-trifluoroethane (HCFC-133a), 1-chloro-1,1-difluoroethane (HCFC-142b), 2-chloro-1,1-difluoroethylene (HCFC-1122), and mixtures thereof;
e. hydrofluorocarbon (HCF) entrainers comprising at least one compound selected from the group consisting of: 1,1,2-trifluoroethylene (HFC-1123), 1-1-difluorothylene (HFC-1132a), 1,1,3,3,3-pentafluoropropene (HFC-1225zc), 1,2,3,3,3-pentafluoropropene (HFC-1225ye), 3,3,3-trifluoropropene (HFC-1243zf), 2,3,3,3-tetrafluoropropene (HFC-1234yf), and mixtures thereof;
f. perfluorocarbon (PFC) entrainers comprising at least one compound selected from the group consisting of: hexafluoroethane (PFC-116), octafluoropropane (PFC-218), 1,1,1,4,4,4-hexafluoro-2-butyne (PFBY-2), hexafluoropropylene (HFP, PFC-1216), hexafluorocyclopropane (PFC-C216), octafluorocyclobutane (PFC-C318), decafluorobutane (PFC-31-10, all isomers), 2,3-dichloro-1,1,1,4,4,4-hexafluoro-2-butene (PFC-1316mxx), octafluoro-2-butene (PFC-1318my, cis and trans), hexafluorobutadiene (PFC-2316), and mixtures thereof.
g. fluoroether entrainers comprising at least one compound selected from the group consisting of: trifluoromethyl-difluoromethyl ether (CF₃OCHF₂, HFOC-125E), 1,1-difluorodimethyl ether, tetrafluorodimetylether (HFOC-134E), difluoromethyl methyl ether (CHF₂OCH₃, HFOC-152aE), pentafluoroethyl methyl ether, and mixtures thereof; and
h. miscellaneous other compounds selected from the group consisting of HFPO, SF₆, chlorine, hexafluoroacetone, PMVE (perfluoromethylvinylether), PEVE (perfluoroethylvinylether), and mixtures thereof.

14. The process of claim 13, wherein the entrainer is chosen from the group consisting of CFC-115 (chloropentafluoroethane), CFC-114 (1,2-dichloro-1,1,2,2-tetrafluoroethane), CFC-1114a (1,1-dichloro-1,2,2,2-tetrafluoroethane), HCFC-21 (dichlorofluoromethane), HCFC-124 (1-chloro-1,2,2,2-tetrafluoroethane), HCFC-124a (1-chloro-1,1,2,2-tetrafluoroethane), HCFC-133a (1-chloro-2,2,2-trifluoroethane), HCFC-142b (1-chloro-1,1-difluoroethane), HCFC-1122 (1-chloro-2,2-difluoroethylene), 1,1,3,3,3-pentafluoropropene (HFC-1225zc), 1,2,3,3,3-pentafluoropropene (HFC-1225ye), 3,3,3- trifluoropropene (HFC-1243zf), 2,3,3,3-tetrafluoropropene (HFC-1234yf),HFC-1123 (trifluoroethylene), PFC-218 (octafluoropropane), PFC-C216 (hexafluorocyclopropane), cis-and trans-PFC-1318 (octafluoro-2-butene), PFC-1216 (hexafluoropropene, HFP), PFC-C318 (octafluorocyclobutane), PFC-31-10my (decafluorobutane), PFC-2316 (hexafluorobutadiene), PEVE (perfluoroethylvinyl ether), PMVE (perfluoromethylvinyl ether), SF₆ (sulfur hexafluoride), Cl₂ (Chlorine), cyclopropane, C₂H₆ (Ethane), propane, n-butane, isobutane, 2,2-dimethylpropane, 1-butene, isobutene, 1,3-butadiene, cis- and trans-2-butene, 1-butyne, vinylacetylene, hexafluoroacetone, 1,1-difluorodimethyl ether, pentafluoroethylmethyl ether, tetrafluorodimethyl ether, and any mixtures thereof.

## Patentansprüche

1. Verfahren zum Trennen einer Mischung, umfassend HF und E-HFC-1234ze, das Verfahren umfassend:
a. Zuführen der Zusammensetzung, umfassend HF und *E*-HFC-1234ze, zu einer ersten Destillationskolonne;
b. Entfernen einer Azeotrop-Zusammensetzung, umfassend HF und *E*-HFC-1234ze, als ein erstes Destillat und entweder i) HF oder ii) *E*-HFC-1234ze als eine Bodensatz-Zusammensetzung der ersten Kolonne;
c. Kondensieren des ersten Destillats, um 2 flüssige Phasen zu bilden, die i) eine HF-reiche Phase und ii) eine *E*-HFC-1234ze-reiche Phase sind; und
d. Rückführen einer ersten flüssigen Phase, angereichert in der gleichen Verbindung, die als Bodensatz der ersten Kolonne entfernt wird, wobei die erste flüssige Phase entweder i) eine HF-reiche Phase oder ii) eine *E*-HFC-1234ze-reiche Phase ist, zurück zu der ersten Destillationskolonne.

2. Verfahren nach Anspruch 1, ferner umfassend Zuführen einer in Schritt (d) nicht rückgeführten zweiten flüssigen Phase, wobei die zweite flüssige Phase entweder i) eine HF-reiche Phase oder ii) eine *E*-HFC-1234ze-reiche Phase ist, zu einer zweiten Destillationskolonne und Zurückgewinnen der in Schritt (b) nicht als die Bodensatz-Zusammensetzung der ersten Kolonne zurückgewonnenen Verbindung als die Bodensatz-Zusammensetzung der zweiten Kolonne.

3. Verfahren nach Anspruch 1 zum Trennen von *E*-HFC-1234ze von einer Mischung, umfassend Fluorwasserstoff und das *E*-HFC-1234ze, wobei das *E*-HFC-1234ze in einer Konzentration vorhanden ist, die größer ist als die Azeotrop-Konzentration für Fluorwasserstoff und das *E*-HFC-1234ze, das Verfahren umfassend:
a. Zuführen der Mischung, umfassend Fluorwasserstoff und das *E*-HFC-1234ze, zu einer ersten Destillationskolonne;
b. Entfernen einer Azeotrop-Zusammensetzung, umfassend Fluorwasserstoff und *E*-HFC-1234ze, als ein erstes Destillat von der ersten Destillationskolonne;
c. Zurückgewinnen von *E*-HFC-1234ze, das im Wesentlichen frei von Fluorwasserstoff ist, aus dem Bodensatz der ersten Destillationskolonne;
d. Kondensieren der Azeotrop-Zusammensetzung, um zwei flüssige Phasen zu bilden, die i) eine Fluorwasserstoff-reiche Phase und ii) eine *E*-HFC-1234ze-reiche Phase sind; und
e. Rückführen der *E*-HFC-1234ze-reichen Phase zu der ersten Destillationskolonne.

4. Verfahren nach Anspruch 3, ferner umfassend:
a. Zuführen der Fluorwasserstoff-reichen Phase zu einer zweiten Destillationskolonne; und
b. Zurückgewinnen von Fluorwasserstoff, der weniger als 100 ppm *E*-HFC-1234ze enthält, aus dem Bodensatz der zweiten Destillationskolonne.

5. Verfahren nach Anspruch 1 zum Trennen von Fluorwasserstoff von einer Mischung, umfassend Fluorwasserstoff und ein *E*-HFC-1234ze, wobei Fluorwasserstoff in einer Konzentration vorhanden ist, die größer ist als die Azeotrop-Konzentration für Fluorwasserstoff und das *E*-HFC-1234ze, das Verfahren umfassend:
a. Zuführen der Mischung, umfassend Fluorwasserstoff und *E*-HFC-1234ze, zu einer ersten Destillationskolonne;
b. Entfernen einer Azeotrop- oder Azeotrop-ähnlichen Zusammensetzung, umfassend *E*-HFC-1234ze und HF, als ein Destillat von der ersten Destillationskolonne;
c. Zurückgewinnen von Fluorwasserstoff, der weniger als 100 ppm *E*-HFC-1234ze enthält, aus dem Bodensatz der ersten Destillationskolonne;
d. Kondensieren der Azeotrop-Zusammensetzung, um zwei flüssige Phasen zu bilden, die eine *E*-HFC-1234ze-reiche Phase und eine Fluorwasserstoff-reiche Phase sind; und
e. Rückführen der Fluorwasserstoff-reichen Phase zu der ersten Destillationskolonne.

6. Verfahren nach Anspruch 5, ferner umfassend:
a. Zuführen der *E*-HFC-1234ze-reichen Phase zu einer zweiten Destillationskolonne; und
b. Zurückgewinnen von *E*-HFC-1234ze, das weniger als 100 ppm Fluorwasserstoff enthält, aus dem Bodensatz der zweiten Destillationskolonne.

7. Verfahren zum Reinigen eines *E*-HFC-1234ze aus einer Mischung, umfassend *E-*HFC-1234ze und HF, wobei das *E*-HFC-1234ze in der Mischung in einer Konzentration vorhanden ist, die größer ist als die Azeotrop-Konzentration für das *E*-HFC-1234ze und HF, das Verfahren umfassend:
a. Hinzufügen eines Schleppmittels zu der Mischung, umfassend *E*-HFC-1234ze und HF, wodurch eine zweite Mischung gebildet wird;
b. Destillieren der zweiten Mischung in einem ersten Destillationsschritt, um eine erste Destillat-Zusammensetzung, umfassend HF, *E*-HFC-1234ze und Schleppmittel, und eine erste Bodensatz-Zusammensetzung, umfassend *E*-HFC-1234ze, zu bilden;
c. Kondensieren der ersten Destillat-Zusammensetzung, um zwei flüssige Phasen zu bilden, die i) eine HF-reiche Phase und ii) eine Schleppmittel-reiche Phase sind; und
d. wahlweises Rückführen der Schleppmittel-reichen Phase zurück zu dem ersten Destillationsschritt.

8. Verfahren zum Reinigen von HF aus einer Mischung, umfassend *E*-HFC-1234ze und HF, wobei HF in einer Konzentration vorhanden ist, die größer ist als die Azeotrop-Konzentration für HF und das *E*-HFC-1234ze, das Verfahren umfassend:
a. Hinzufügen eines Schleppmittels zu der Mischung, umfassend *E*-HFC-1234ze und HF, wodurch eine zweite Mischung gebildet wird;
b. Destillieren der zweiten Mischung in einem ersten Destillationsschritt, um eine erste Destillat-Zusammensetzung, umfassend HF, Schleppmittel und *E*-HFC-1234ze, und eine erste Bodensatz-Zusammensetzung, umfassend HF, zu bilden;
c. Kondensieren der ersten Destillat-Zusammensetzung, um zwei flüssige Phasen zu bilden, die i) eine Schleppmittel-reiche Phase und ii) eine HF-reiche Phase sind; und
d. wahlweises Rückführen der HF-reichen Phase zurück zu dem ersten Destillationsschritt.

9. Verfahren nach Anspruch 8, ferner umfassend Zuführen der Schleppmittel-reichen Phase von Schritt (c) zu einem zweiten Destillationsschritt und Bilden einer zweiten Destillat-Zusammensetzung, umfassend ein Azeotrop von Schleppmittel und HF, und einer zweiten Bodensatz-Zusammensetzung, umfassend HF, der weniger als 100 ppm Schleppmittel enthält.

10. Verfahren zum Trennen von *E*-HFC-1234ze von einer Mischung von *E*-HFC-1234ze, HF und mindestens einem von HFC-245fa oder HFC-245eb, das Verfahren umfassend:
a) Unterziehen der Mischung einem ersten Destillationsschritt, wobei zusätzliches *E*-HFC-1234ze aus einem zweiten Destillationsschritt zugeführt wird, um ein erstes Destillat, umfassend ein Azeotrop von *E*-HFC-1234ze und HF, und eine erste Bodensatz-Zusammensetzung, umfassend mindestens eines von HFC-245fa oder HFC-245eb, zu bilden;
b) Zuführen des ersten Destillats zu einem zweiten Destillationsschritt, um ein zweites Destillat, umfassend ein Azeotrop von *E*-HFC-1234ze und HF, und eine zweite Bodensatz-Zusammensetzung, umfassend *E*-HFC-1234ze, das weniger als 100 ppm HF enthält, zu bilden;
c) Kondensieren des zweiten Destillats, um zwei flüssige Phasen zu bilden, die i) eine HF-reiche Phase und ii) eine *E*-HFC-1234ze-reiche Phase sind; und
d) Rückführen der *E*-HFC-1234ze-reichen Phase aus (c) zurück zu dem ersten Destillationsschritt.

11. Verfahren zum Trennen von HF von einer Mischung, umfassend *E*-HFC-1234ze, HF und mindestens eines von HFC-245fa oder HFC-245eb, das Verfahren umfasst:
a. Hinzufügen eines Schleppmittels zu der Mischung, umfassend *E*-HFC-1234ze, HF und mindestens eines von HFC-245fa oder HFC-245eb, wodurch eine zweite Mischung gebildet wird;
b. Destillieren der zweiten Mischung in einem ersten Destillationsschritt, um eine erste Destillat-Zusammensetzung, umfassend HF und Schleppmittel, und eine erste Bodensatz-Zusammensetzung, umfassend *E*-HFC-1234ze und mindestens eines von HFC-245fa oder HFC-245eb, zu bilden;
c. Kondensieren der ersten Destillat-Zusammensetzung, um zwei flüssige Phasen zu bilden, die (i) eine Schleppmittel-reiche Phase und (ii) eine HF-reiche Phase sind; und
d. Rückführen der Schleppmittel-reichen Phase zurück zu dem ersten Destillationsschritt.

12. Verfahren nach Anspruch 11, ferner umfassend das Rückführen der zweiten Destillat-Zusammensetzung zurück zu den beiden flüssigen Phasen.

13. Verfahren nach Anspruch 7, 8 oder 11, wobei das Schleppmittel aus der Gruppe ausgewählt wird, bestehend aus:
a. Kohlenwasserstoff-Schleppmitteln, umfassend mindestens eine Verbindung, ausgewählt aus der Gruppe, bestehend aus: Methan, Ethan, Ethylen, Acetylen, Vinylacetylen, n-Propan, Propylen, Propyn, Cyclopropan, Cyclopropen, Propadien, n-Butan, Isobutan, 1-Buten, Isobuten, 1,3-Butadien, 2,2-Dimethylpropan, cis-2-Buten, trans-2-Buten, 1-Butyn, n-Pentan, Isopentan, Neopentan, Cyclopentan, 1-Penten, 2-Penten und Mischungen davon;
b. Chlorkohlenwasserstoff-Schleppmitteln, ausgewählt aus der Gruppe, bestehend aus Methylenchlorid, Methylchlorid und Mischungen davon;
c. Fluorchlorkohlenwasserstoff- bzw. CFC-Schleppmitteln, umfassend mindestens eine Verbindung, ausgewählt aus der Gruppe, bestehend aus: Dichlordifluormethan (CFC-12), 2-Chlor-1,1,2-trifluorethylen, Chlorpentafluorethan (CFC-115), 1,2-Dichlor-1,1,2,2-tetrafluorethan (CFC-114), 1,1-Dichlor-1,2,2,2-tetrafluorethan (CFC-114a), 1,1,2-Trichlor-1,2,2-trifluorethan (CFC-113), 1,1,1-Trichlor-2,2,2-trifluorethan (CFC-113a), 1,1,2-Trichlor-1,2,3,3,3-pentafluorpropan (CFC-215bb), 2,2-Dichlor-1,1,1,3,3,3-hexafluorpropan (CFC-216aa), 1,2-Dichlor-1,1,2,3,3,3-hexafluorpropan (CFC-216ba), 2-Chlor-1,1,1,2,3,3,3-heptafluorpropan (CFC-217ba), 2-Chlor-1,1,3,3,3-pentafluorpropen (CFC-1215xc) und Mischungen davon;
d. Hydrochlorfluorcarbon- bzw. HCFC-Schleppmitteln, umfassend mindestens eine Verbindung, ausgewählt aus der Gruppe, bestehend aus: Dichlorfluormethan (HCFC-21), 1,1-Dichlor-3,3,3-trifluorethan (HCFC-123), 1,1-Dichlor-1-fluorethan (HCFC-141b), 2-Chlor-1,1,1,2-tetrafluorethan (HCFC-124), 1-Chlor-1,1,2,2-tetrafluorethan (HCFC-124a), 2-Chlor-1,1,1-trifluorethan (HCFC-133a), 1-Chlor-1,1-difluorethan (HCFC-142b), 2-Chlor-1,1-difluorethylen (HCFC-1122) und Mischungen davon;
e. Hydrofluorcarbon- bzw. HFC-Schleppmitteln, umfassend mindestens eine Verbindung, ausgewählt aus der Gruppe, bestehend aus: 1,1,2-Trifluorethylen (HFC-1123), 1,1-Difluorethylen (HFC-1132a), 1,1,3,3,3-Pentafluorpropen (HFC-1225zc), 1,2,3,3,3-Pentafluorpropen (HFC-1225ye), 3,3,3-Trifluorpropen (HFC-1243zf), 2,3,3,3-Tetrafluorpropen (HFC-1234yf) und Mischungen davon;
f. Perfluorcarbon- bzw. PFC-Schleppmitteln, umfassend mindestens eine Verbindung, ausgewählt aus der Gruppe, bestehend aus: Hexafluorethan (PFC-116), Octafluorpropan (PFC-218), 1,1,1,4,4,4-Hexafluor-2-butin (PFBY-2), Hexafluorpropylen (HFP, (PFC-1216), Hexafluorcyclopropan (PFC-C216), Octafluorcyclobutan (PFC-C318), Decafluorbutan (PFC-31-10, alle Isomere), 2,3-Dichlor-1,1,1,4,4,4-hexafluor-2-buten (PFC-1316mxx), Octafluor-2-buten (PFC-1318my, cis und trans), Hexafluorbutadien (PFC-2316) und Mischungen davon;
g. Fluorether-Schleppmitteln, umfassend mindestens eine Verbindung, ausgewählt aus der Gruppe, bestehend aus: Trifluormethyl-difluormethylether (CF₃OCHF₂, HFOC-125E), 1,1-Difluordimethylether, Tetrafluordimethylether (HFOC-134E), Difluormethylmethylether (CHF₂OCH₃, HFOC-152aE), Pentafluorethylmethylether und Mischungen davon; und
h. verschiedene andere Verbindungen, ausgewählt aus der Gruppe, bestehend aus HFPO, SF₆, Chlor, Hexafluoraceton, PMVE (Perfluormethylvinylether), PEVE (Perfluorethylvinylether) und Mischungen davon.

14. Verfahren nach Anspruch 13, wobei das Schleppmittel aus der Gruppe ausgewählt ist, bestehend aus CFC-115 (Chlorpentafluorethan), CFC-114 (1,2-Dichlor-1,1,2,2-tetrafluorethan), CFC-114a (l,l-Dichlor-1,2,2,2-tetrafluorethan), HCFC-21 (Dichlorfluormethan), HCFC-124 (1-Chlor-1,2,2,2-tetrafluorethan), HCFC-124a (1-Chlor-1,1,2,2-tetrafluorethan), HCFC-133a (1-Chlor-2,2,2-trifluorethan), HCFC-142b (1-Chlor-1,1-difluorethan), HCFC-1122 (1-Chlor-2,2-Difluorethylen), 1,1,3,3,3-Pentafluorpropen (HFC-1225zc), 1,2,3,3,3-Pentafluorpropen (HFC-1225ye), 3,3,3-Trifluorpropen (HFC-1243zf), 2,3,3,3-Tetrafluorpropen (HFC-1234yf), HFC-1123 (Trifluorethylen), PFC-218 (Octafluorpropan), PFC-C216 (Hexafluorcyclopropan), cis-und trans-PFC-1318 (Octafluor-2-buten), PFC-1216 (Hexafluorpropen, HFP), PFC-C318 (Octafluorcyclobutan), PFC-31-10my (Decafluorbutan), PFC-2316 (Hexafluorbutadien), PEVE (Perfluorethylvinylether), PMVE (Perfluormethylvinylether), SF₆ (Schwefelhexafluorid), Cl₂ (Chlor), Cyclopropan, C₂H₆ (Ethan), Propan, n-Butan, Isobutan, 2,2-Dimethylpropan, 1-Buten, Isobuten, 1,3-Butadien, cis- und trans-2-Buten, 1-Butyn, Vinylacetylen, Hexafluoraceton, 1,1-Difluordimethylether, Pentafluorethylmethylether, Tetrafluordimethylether und beliebige Mischungen davon.

## Revendications

1. Procédé pour séparer un mélange comprenant HF et *E*-HFC-1234ze, ledit procédé comprenant:
a. l'alimentation de la composition comprenant HF et *E*-HFC-1234ze à une première colonne de distillation;
b. le retrait d'une composition d'azéotrope comprenant HF et *E*-HFC-1234ze en tant que premier distillat et de soit i) HF soit ii) *E*-HFC-1234ze en tant que composition de produit de queue de la première colonne;
c. la condensation du premier distillat pour former 2 phases liquides, qui sont i) une phase riche en HF et ii) une phase riche en *E*-HFC-1234ze; et
d. le recyclage d'une première phase liquide enrichie en le même composé qui est retiré en tant que produit de queue de la première colonne, ladite première phase liquide étant soit i) une phase riche en HF soit ii) une phase riche en *E*-HFC-1234ze, dans la première colonne de distillation.

2. Procédé selon la revendication 1, comprenant en outre l'alimentation d'une seconde phase liquide non recyclée dans l'étape (d), ladite seconde phase liquide étant soit i) une phase riche en HF soit ii) une phase riche en *E*-HFC-1234ze, à une seconde colonne de distillation, et la récupération du composé non récupéré dans l'étape (b) en tant que composition de produit de queue de la première colonne en tant que composition de produit de queue de la seconde colonne.

3. Procédé selon la revendication 1 pour séparer *E*-HFC-1234ze à partir d'un mélange comprenant du fluorure d'hydrogène et ledit *E*-HFC-1234ze, dans lequel ledit *E-*HFC-1234ze est présent en une concentration supérieure à la concentration d'azéotrope pour le fluorure d'hydrogène et ledit *E*-HFC-1234ze, ledit procédé comprenant:
a. l'alimentation dudit mélange comprenant du fluorure d'hydrogène et ledit *E*-HFC-1234ze à une première colonne de distillation;
b. le retrait d'une composition d'azéotrope comprenant du fluorure d'hydrogène et *E*-HFC-1234ze en tant que premier distillat de la première colonne de distillation;
c. la récupération du *E*-HFC-1234ze essentiellement exempt de fluorure d'hydrogène à partir du produit de queue de la première colonne de distillation;
d. la condensation de la composition d'azéotrope pour former deux phases liquides, qui sont i) une phase riche en fluorure d'hydrogène et ii) une phase riche en *E-*HFC-1234ze; et
e. le recyclage de la phase riche en *E*-HFC-1234ze à la première colonne de distillation.

4. Procédé selon la revendication 3, comprenant en outre:
a. l'alimentation de la phase riche en fluorure d'hydrogène à une seconde colonne de distillation, et
b. la récupération du fluorure d'hydrogène contenant moins de 100ppm de *E-*HFC-1234ze à partir du produit de queue de la seconde colonne de distillation.

5. Procédé selon la revendication 1 pour séparer le fluorure d'hydrogène à partir d'un mélange comprenant du fluorure d'hydrogène et un *E*-HFC-1234ze, dans lequel le fluorure d'hydrogène est présent en une concentration supérieure à la concentration d'azéotrope pour le fluorure d'hydrogène et ledit *E*-HFC-1234ze, ledit procédé comprenant:
a. l'alimentation dudit mélange comprenant du fluorure d'hydrogène et *E-*HFC-1234ze à une première colonne de distillation;
b. le retrait d'une composition d'azéotrope ou de type azéotrope comprenant *E*-HFC-1234ze et HF en tant que distillat de la première colonne de distillation;
c. la récupération du fluorure d'hydrogène contenant moins de 100ppm de *E-*HFC-1234ze à partir du produit de queue de la première colonne de distillation;
d. la condensation de la composition d'azéotrope pour former deux phases liquides, qui sont une phase riche en *E*-HFC-1234ze et une phase riche en fluorure d'hydrogène; et
e. le recyclage de la phase riche en HF à la première colonne de distillation.

6. Procédé selon la revendication 5, comprenant en outre:
a. l'alimentation de la phase riche en *E*-HFC-1234ze à une seconde colonne de distillation; et
b. la récupération du *E*-HFC-1234ze contenant moins de 100ppm de fluorure d'hydrogène à partir du produit de queue de la seconde colonne de distillation.

7. Procédé pour la purification d'un *E*-HFC-1234ze à partir d'un mélange comprenant *E*-HFC-1234ze et HF, dans lequel ledit *E*-HFC-1234ze est présent dans ledit mélange en une concentration supérieure à la concentration d'azéotrope pour lesdits *E*-HFC-1234ze et HF, ledit procédé comprenant:
a. l'addition d'un agent d'entraînement au mélange comprenant *E*-HFC-1234ze et HF, formant ainsi un second mélange;
b. la distillation dudit second mélange dans une première étape de distillation pour former une première composition de distillat comprenant HF, *E*-HFC-1234ze et l'agent d'entraînement, et une première composition de produit de queue comprenant *E-*HFC-1234ze;
c. la condensation de ladite première composition de distillat pour former deux phases liquides, qui sont i) une phase riche en HF et ii) une phase riche en agent d'entraînement; et
d. optionnellement, le recyclage de la phase riche en agent d'entraînement dans la première étape de distillation.

8. Procédé pour la purification de HF à partir d'un mélange comprenant un *E*-HFC-1234ze et HF, dans lequel HF est présent en une concentration supérieure à la concentration d'azéotrope pour HF et ledit *E*-HFC-1234ze, ledit procédé comprenant:
a. l'addition d'un agent d'entraînement au mélange comprenant *E*-HFC-1234ze et HF, formant ainsi un second mélange;
b. la distillation dudit second mélange dans une première étape de distillation pour former une première composition de distillat comprenant un HF, l'agent d'entraînement et *E*-HFC-1234ze, et une première composition de produit de queue comprenant HF;
c. la condensation de ladite première composition de distillat pour former deux phases liquides, qui sont i) une phase riche en agent d'entraînement et ii) une phase riche en HF; et
d. optionnellement, le recyclage de la phase riche en HF dans la première étape de distillation.

9. Procédé selon la revendication 8, comprenant en outre l'alimentation de la phase riche en agent d'entraînement de l'étape (c) à une seconde étape de distillation et la formation d'une seconde composition de distillat comprenant un azéotrope de l'agent d'entraînement et de HF et d'une seconde composition de produit de queue comprenant un HF contenant moins de 100ppm d'agent d'entraînement.

10. Procédé pour la séparation de *E*-HFC-1234ze à partir d'un mélange de *E*-HFC-1234ze, de HF et d'au moins l'un parmi HFC-245fa ou HFC-245eb, ledit procédé comprenant:
a) la soumission dudit mélange à une première étape de distillation, dans laquelle du *E*-HFC-1234ze supplémentaire est alimenté à partir d'une seconde étape de distillation, pour former un premier distillat comprenant un azéotrope de *E*-HFC-1234ze et de HF et une première composition de produit de queue comprenant au moins l'un parmi HFC-245fa ou HFC-245eb;
b) l'alimentation dudit premier distillat à une seconde étape de distillation pour former un second distillat comprenant un azéotrope de *E*-HFC-1234ze et de HF et une seconde composition de produit de queue comprenant un *E*-HFC-1234ze contenant moins de 100ppm de HF;
c) la condensation dudit second distillat pour former deux phases liquides, qui sont i) une phase riche en HF et ii) une phase riche en *E*-HFC-1234ze; et
d) le recyclage de la phase riche en *E*-HFC-1234ze de (c) dans la première étape de distillation.

11. Procédé pour séparer HF à partir d'un mélange comprenant *E*-HFC-1234ze, HF et au moins l'un parmi HFC-245fa et HFC-245eb, ledit procédé comprenant:
a. l'addition d'un agent d'entraînement au mélange comprenant *E*-HFC-1234ze, HF et au moins l'un parmi HFC-245fa et HFC-245eb, formant ainsi un second mélange;
b. la distillation dudit second mélange dans une première étape de distillation pour former une première composition de distillat comprenant HF et un agent d'entraînement et une première composition de produit de queue comprenant *E*-HFC-1234ze et au moins l'un parmi HFC-245fa ou HFC-245eb;
c. la condensation de ladite première composition de distillat pour former deux phases liquides, qui sont (i) une phase riche en agent d'entraînement et (ii) une phase riche en HF; et
d. le recyclage de la phase riche en agent d'entraînement dans la première étape de distillation.

12. Procédé selon la revendication 11, comprenant en outre le recyclage de ladite seconde composition de distillat dans les deux phases liquides.

13. Procédé selon la revendication 7, 8 ou 11, dans lequel ledit agent d'entraînement est choisi dans le groupe constitué par:
a. les agents d'entraînement hydrocarbonés comprenant au moins un composé choisi dans le groupe constitué par: méthane, éthane, éthylène, acétylène, vinylacétylène, n-propane, propylène, propyne, cyclopropane, cyclopropène, propadiène, n-butane, isobutane, 1-butène, isobutène, 1,3-butadiène, 2,2-diméthylpropane, cis-2-butène, trans-2-butène, 1-butyne, n-pentane, isopentane, néopentane, cyclopentane, 1-pentène, 2-pentène, et les mélanges de ceux-ci;
b. les agents d'entraînement chlorocarbonés choisis dans le groupe constitué par le chlorure de méthylène, le chlorure de méthyle et les mélanges de ceux-ci;
c. les agents d'entaînement chlorofluorocarbonés (CFC) comprenant au moins un composé choisi dans le groupe constitué par: dichlorodifluorométhane (CFC-12), 2-chloro-1,1,2-trifluoroéthylène, chloropentafluoroéthane (CFC-115), 1,2-dichloro-1,1,2,2-tétrafluoroéthane (CFC-114), 1,1-dichloro-1,2,2,2-tétrafluoroéthane (CFC-114a), 1,1,2-trichloro-1,2,2-trifluoroéthane (CFC-113), 1,1,1-trichloro-2,2,2-trifluoroéthane (CFC-113a), 1,1,2-trichloro-1,2,3,3,3-pentafluoropropane (CFC-215bb), 2,2-dichloro-1,1,1,3,3,3-hexafluoropropane (CFC-216aa), 1,2-dichloro-1,1,2,3,3,3-hexafluoropropane (CFC-216ba), 2-chloro-1,1,1,2,3,3,3-heptafluoropropane (CFC-217ba), 2-chloro-1,1,3,3,3-pentafluoropropène (CFC-1215xc), et les mélanges de ceux-ci;
d. les agents d'entraînement hydrochlorofluorocarbonés (HCFC) comprenant au moins un composé choisi dans le groupe constitué par: dichlorofluorométhane (HCFC-21), 1,1-dichloro-3,3,3-trifluoroéthane (HCFC-123), 1,1-dichloro-1-fluoroéthane (HCFC-141b), 2-chloro-1,1,1,2-tétrafluoroéthane (HCFC-124), 1-chloro-1,1,2,2-tétrafluoroéthane (HCFC-124a), 2-chloro-1,1,1-trifluoroéthane (HCFC-133a), 1-chloro-1,1-difluoroéthane (HCFC-142b), 2-chloro-1,1-difluoroéthylène (HCFC-1122), et les mélanges de ceux-ci;
e. les agents d'entraînement hydrofluorocarbonés (HFC) comprenant au moins un composé choisi dans le groupe constitué par: 1,1,2-trifluoroéthylène (HFC-1123), 1,1-difluoroéthylène (HFC-1132a), 1,1,3,3,3-pentafluoropropène (HFC-1225zc), 1,2,3,3,3-pentafluoropropène (HFC-1225ye), 3,3,3-trifluoropropène (HFC-1243zf), 2,3,3,3-tétrafluoropropène (HFC-1234yf), et les mélanges de ceux-ci;
f. les agents d'entraînement perfluorocarbonés (PFC) comprenant au moins un composé choisi dans le groupe constitué par: hexafluoroéthane (PFC-116), octafluoropropane (PFC-218), 1,1,1,4,4,4-hexafluoro-2-butyne (PFBY-2), hexafluoropropylène (HFP, PFC-1216), hexafluorocyclopropane (PFC-C216), octafluorocyclobutane (PFC-C318), décafluorobutane (PFC-31-10, tous isomères), 2,3-dichloro-1,1,1,4,4,4-hexafluoro-2-butène (PFC-1316mxx), octafluoro-2-butène (PFC-1318my, cis et trans), hexafluorobutadiène (PFC-2316), et les mélanges de ceux-ci;
g. les agents d'entraînement fluoroéthers comprenant au moins un composé choisi dans le groupe constitué par: éther trifluorométhyl-difluorométhylique (CF₃OCHF₂, HFOC-125E), éther 1,1-difluorodiméthylique, éther tétrafluorodiméthylique (HFOC-134E), éther difluorométhyl méthylique (CHF₂OCH₃, HFOC-152aE), éther pentafluoroéthyl méthylique, et les mélanges de ceux-ci; et
h. divers autres composés choisis dans le groupe constitué par HFPO, SF₆, chlore, hexafluoroacétone, PMVE (éther perfluorométhylvinylique), PEVE (éther perfluoroéthylvinylique), et les mélanges de ceux-ci.

14. Procédé selon la revendication 13, dans lequel l'agent d'entraînement est choisi dans le groupe constitué par CFC-115 (chloropentafluoroéthane), CFC-114 (1,2-dichloro-1,1,2,2-tétrafluoroéthane), CFC-114a (1,1-dichloro-1,2,2,2-tétrafluoroéthane), HCFC-21 (dichlorofluorométhane), HCFC-124 (1-chloro-1,2,2,2-tétrafluoroéthane), HCFC-124a (1-chloro-1,1,2,2-tétrafluoroéthane), HCFC-133a (1-chloro-2,2,2-trifluoroéthane), HCFC-142b (1-chloro-1,1-difluoroéthane), HCFC-1122 (1-chloro-2,2-difluoroéthylène), 1,1,3,3,3-pentafluoropropène (HFC-1225zc), 1,2,3,3,3-pentafluoropropène (HFC-1225ye), 3,3,3-trifluoropropène (HFC-1243zf), 2,3,3,3-tétrafluoropropène (HFC-1234yf), HFC-1123 (trifluoroéthylène), PFC-218 (octafluoropropane), PFC-C216 (hexafluorocyclopropane), cis- et trans-PFC-1318 (octafluoro-2-butène), PFC-1216 (hexafluoropropène, HFP), PFC-C318 (octafluorocyclobutane), PFC-31-10my (décafluorobutane), PFC-2316 (hexafluorobutadiène), PEVE (éther perfluoroéthylvinylique), PMVE (éther perfluorométhylvinylique), SF₆ (hexafluorure de soufre), Cl₂ (chlore), cyclopropane, C₂H₆ (éthane), propane, n-butane, isobutane, 2,2-diméthylpropane, 1-butène, isobutène, 1,3-butadiène, cis- et trans-2-butène, 1-butyne, vinylacétylène, hexafluoroacétone, éther 1,1-difluorodiméthylique, éther pentafluoroéthylméthylique, éther tétrafluorodiméthylique, et tous mélanges de ceux-ci.
